(19) Europäisches Patentamt
European Patent Office
Office européen des brevets

(11)    **EP 3 297 705 B1**

(12)    **EUROPEAN PATENT SPECIFICATION**

(45) Date of publication and mention
of the grant of the patent:
**24.06.2026   Bulletin 2026/26**

(21) Application number: **16725722.9**

(22) Date of filing: **18.05.2016**

(51) International Patent Classification (IPC):
*A61M 5/142* (2006.01)      *G16H 20/17* (2018.01)
*G16H 40/67* (2018.01)      *A61M 5/172* (2006.01)

(52) Cooperative Patent Classification (CPC):
**A61M 5/1723; A61M 5/14244; G16H 20/17;**
**G16H 40/67;** A61M 2005/14208; A61M 2005/14296;
A61M 2205/502

(86) International application number:
**PCT/US2016/033137**

(87) International publication number:
**WO 2016/187342 (24.11.2016 Gazette 2016/47)**

(54) **INFUSION DEVICES FOR THERAPY RECOMMENDATIONS BASED ON RESIDUAL AMOUNT OF ACTIVE FLUID IN THE BODY**

INFUSIONSGERÄT FÜR THERAPIEEMPFEHLUNGEN AUF BASIS DER RESTMENGE AN AKTIVER FLÜSSIGKEIT IM KÖRPER

DISPOSITIF DE PERFUSION POUR RECOMMANDATION DE THÉRAPIE SUR LA BASE DE LA QUANTITÉ RÉSIDUELLE DE FLUIDE ACTIF DANS LE CORPS

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:  **20.05.2015   US 201514717902**

(43) Date of publication of application:
**28.03.2018   Bulletin 2018/13**

(73) Proprietor: **Medtronic MiniMed, Inc.**
**Northridge, CA 91325-1219 (US)**

(72) Inventors:
• **YAN, Jin**
**Chatsworth, California 91311 (US)**
• **MONIRABBASI, Salman**
**Playa Vista, California 90094 (US)**
• **NAYLOR, Ross H.**
**Fullerton, California 92835 (US)**
• **PALERM, Cesar C.**
**Pasadena, California 91101 (US)**
• **LINTEREUR, Louis J.**
**Stevenson Ranch, California 91381 (US)**
• **SOKOLOVSKYY, Dmytro Y.**
**Moorpark, CA 93021 (US)**

(74) Representative: **Zimmermann & Partner**
**Patentanwälte mbB**
**P.O. Box 330 920**
**80069 München (DE)**

(56) References cited:
EP-A1- 3 154 607          WO-A1-2015/191459
WO-A2-2009/095908      WO-A2-2009/133558
US-A1- 2010 094 251      US-A1- 2010 295 686
US-A1- 2010 298 685      US-A1- 2014 066 888
US-A1- 2016 158 441

**Description**

TECHNICAL FIELD

Embodiments of the subject matter described herein relate generally to medical devices, and more particularly, embodiments of the subject matter relate to providing therapy information to a user during operation of a fluid infusion device.

BACKGROUND

Infusion pump devices and systems are relatively well known in the medical arts, for use in delivering or dispensing an agent, such as insulin or another prescribed medication, to a patient. A typical infusion pump includes a pump drive system which typically includes a small motor and drive train components that convert rotational motor motion to a translational displacement of a plunger (or stopper) in a reservoir that delivers medication from the reservoir to the body of a user via a fluid path created between the reservoir and the body of a user. Use of infusion pump therapy has been increasing, especially for delivering insulin for diabetics.

Continuous insulin infusion provides greater control of a diabetic's condition, and hence, control schemes are being developed that allow insulin infusion pumps to monitor and regulate a user's blood glucose level in a substantially continuous and autonomous manner. Regulating blood glucose level is complicated by variations in the response time for the type of insulin being used along with variations in a user's individual insulin response and daily activities (e.g., exercise, carbohydrate consumption, bolus administration, and the like). To compensate for these variations, the amount of insulin being infused in an automated manner may also vary. However, this poses challenges when transitioning from an automated delivery control mode to a more manually-intensive delivery mode where the user desires information or feedback for manually regulating his or her blood glucose level, such as, for example, current amount of active insulin delivered that is still to be metabolized. While techniques exist for calculating active insulin based on manual correction boluses or meal boluses, many current approaches do not accurately account for variable basal deliveries when they are the primary source of insulin or provide a way for the user to conveniently gauge the amount of active insulin.
WO 2015/191459 relates to insulin delivery systems and methods
US 2010/0295686 A1 relates to usability features for integrated insulin delivery systems.

US2010/0094251 discloses an infusion pump system configured to provide alarms in response to a user's blood glucose and insulin characteristics. The user's total insulin load is also determined. WO2009/133558 discloses a method for selecting bolus delivery patterns by depicting carbohydrate intake as a multi-dimensional space. US2016/0158441 discloses an infusion pump system having a plurality of operating modes including a closed-loop control mode.

BRIEF SUMMARY

Infusion systems, infusion devices, and related operating methods are provided. The invention is defined by claims 1, 2 and 11 and preferred embodiments are defined by the dependent claims. An embodiment of a method of operating an infusion device to deliver fluid to a body of a user is provided. The method involves autonomously operating the infusion device to deliver a variable rate of infusion of the fluid in a first operating mode and determining a residual amount of active fluid in the body of the user based at least in part on the variable rate of infusion delivered by the infusion device in the first operating mode. In response to identifying a change in operating mode from the first operating mode, the method generates a user notification based at least in part on the residual amount of active fluid.

In another embodiment, a method of operating an infusion device to deliver insulin to a body of a user involves operating the infusion device in a closed-loop operating mode to autonomously deliver a variable rate of infusion to the body based on a difference between glucose measurements from the body of the user and a reference glucose value, recursively determining a total active insulin amount based at least in part on the variable rate of infusion, and recursively determining a nominal active insulin amount based at least in part on a constant rate of infusion. Upon exiting the closed-loop operating mode, the method determines a residual active insulin amount based on a difference between the total active insulin amount and the nominal active insulin amount and displays a graphical user notification influenced by the residual active insulin amount.

An embodiment of an infusion system is also provided, The infusion system includes a user interface, an infusion device including a motor operable to deliver fluid to a body of a user and a control system coupled to the motor, and a sensing arrangement to obtain measurement values for a physiological condition influenced by the fluid from the body of the user. The control system is coupled to the user interface and the sensing arrangement to autonomously operate the motor to deliver a variable basal rate of infusion based on the measurement values, determine a residual amount of the fluid that is active in the body of the user based at least in part on the variable basal rate of infusion, and provide a user notification on the user interface that is influenced by the residual amount.

This summary is provided to introduce a selection of concepts in a simplified form that are further described below in the detailed description. This summary is not intended to identify key features or essential features of the claimed subject-matter.

BRIEF DESCRIPTION OF THE DRAWINGS

A more complete understanding of the subject matter may be derived by referring to the detailed description and claims when considered in conjunction with the following figures, wherein like reference numbers refer to similar elements throughout the figures, which may be illustrated for simplicity and clarity and are not necessarily drawn to scale.

FIG. 1 depicts an exemplary embodiment of an infusion system;

FIG. 2 depicts a plan view of an exemplary embodiment of a fluid infusion device suitable for use in the infusion system of FIG. 1;

FIG. 3 is an exploded perspective view of the fluid infusion device of FIG. 2;

FIG. 4 is a cross-sectional view of the fluid infusion device of FIGS. 2-3 as viewed along line 4-4 in FIG. 3 when assembled with a reservoir inserted in the infusion device;

FIG. 5 is a block diagram of an exemplary control system suitable for use in a fluid infusion device, such as the fluid infusion device of FIG. 1 or FIG. 2;

FIG. 6 is a block diagram of an exemplary pump control system suitable for use in the control system of FIG. 5;

FIG. 7 is a block diagram of a closed-loop control system that may be implemented or otherwise supported by the pump control system in the fluid infusion device of FIG. 5 in one or more exemplary embodiments; and

FIG. 8 is a flow diagram of an exemplary active insulin notification process suitable for use with the control system of FIG. 5 in one or more exemplary embodiments.

DETAILED DESCRIPTION

The following detailed description is merely illustrative in nature and is not intended to limit the embodiments of the subject matter or the application and uses of such embodiments. As used herein, the word "exemplary" means "serving as an example, instance, or illustration." Any implementation described herein as exemplary is not necessarily to be construed as preferred or advantageous over other implementations. Furthermore, there is no intention to be bound by any expressed or implied theory presented in the preceding technical field, background, brief summary or the following detailed description.

While the subject matter described herein can be implemented in any electronic device that includes a motor, exemplary embodiments described below are implemented in the form of medical devices, such as portable electronic medical devices. Although many different applications are possible, the following description focuses on a fluid infusion device (or infusion pump) as part of an infusion system deployment. For the sake of brevity, conventional techniques related to infusion system operation, insulin pump and/or infusion set operation, and other functional aspects of the systems (and the individual operating components of the systems) may not be described in detail here. Examples of infusion pumps may be of the type described in, but not limited to, United States Patent numbers: 4,562,751; 4,685,903; 5,080,653; 5,505,709; 5,097,122; 6,485,465; 6,554,798; 6,558,320; 6,558,351; 6,641,533; 6,659,980; 6,752,787; 6,817,990; 6,932,584; and 7,621,893.

Embodiments of the subject matter described herein generally relate to fluid infusion devices including a motor that is operable to linearly displace a plunger (or stopper) of a reservoir provided within the fluid infusion device to deliver a dosage of fluid, such as insulin, to the body of a user. Dosage commands that govern operation of the motor may be generated in an automated manner in accordance with the delivery control scheme associated with a particular operating mode, and the dosage commands may be generated in a manner that is influenced by a current (or most recent) measurement of a physiological condition in the body of the user. For example, in a closed-loop operating mode, dosage commands may be generated based on a difference between a current (or most recent) measurement of the interstitial fluid glucose level in the body of the user and a target (or reference) glucose value. In this regard, the rate of infusion may vary as the difference between a current measurement value and the target measurement value fluctuates. For purposes of explanation, the subject matter is described herein in the context of the infused fluid being insulin for regulating a glucose level of a user (or patient); however, it should be appreciated that many other fluids may be administered through infusion, and the subject matter described herein is not necessarily limited to use with insulin.

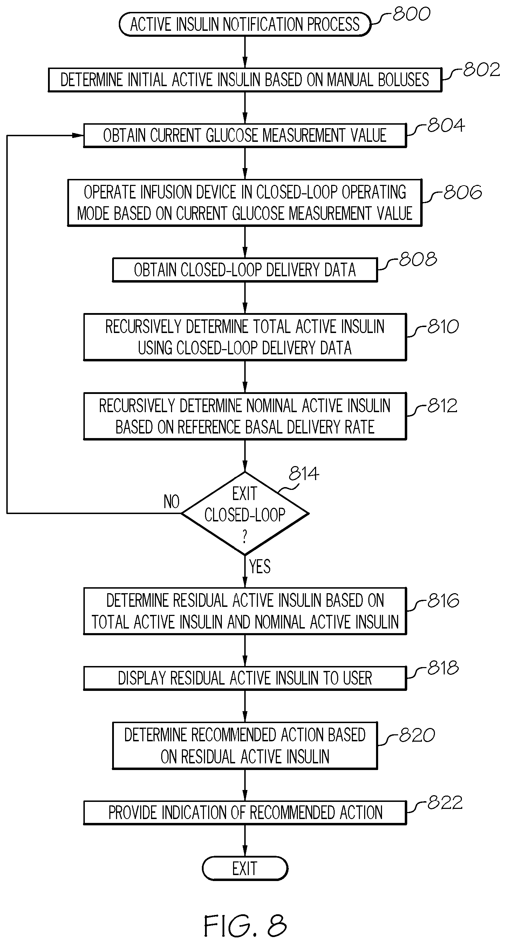

As described in greater detail below, primarily in the context of FIG. 8, in exemplary embodiments described herein, a residual amount of active insulin in the body of a patient is determined based at least in part on the variable basal rate of infusion delivered by the infusion device in an autonomous operating mode. The residual amount of insulin represents the remaining portion of the infused insulin yet to be metabolized that exceeds a nominal amount of active insulin corresponding to a reference basal rate of infusion for maintaining the patient's physiological condition at or near a desired level. To determine the residual amount of active insulin, the current total amount of active insulin is recursively calculated based on the variable basal rate of infusion dictated by the current operating mode, any manually-initiated correction boluses or meal boluses, and preceding values for the amount of active insulin at the preceding sampling time. The nominal amount of active insulin is also recursively calculated based on a reference basal rate of infusion and preceding values for the nominal amount of active insulin. In exemplary embodiments, the reference basal rate is based on a patient-specific total daily dose value, which approximates or otherwise represents the total amount of insulin required to be delivered on a daily basis to maintain the patient's glucose level at a target glucose value or within a desired range of glucose values. The current residual amount of active insulin then corresponds to the difference between the current total amount of active insulin and the nominal amount of active insulin. Thus, the residual active insulin amount represents the portion of the total amount of active insulin in excess of the expected amount of active amount of active insulin for regulating the patient's glucose level to a desired fasting glucose level.

In response to identifying a change in operating mode (e.g., transitioning from the automated operating mode to a more manually-intensive operating mode), the residual amount of active insulin is utilized to automatically generate one or more graphical indications or notifications for the patient, which, in turn, may be utilized by the patient in determining how to manually control his or her therapy going forward. For example, the current residual active insulin may be presented or otherwise displayed to the patient for manual assessment. By accounting for variable basal delivery rates and the patient's total daily dose, the residual amount of active insulin provides a more accurate representation of the patient's current and future glycemic state relative to traditional insulin-on-board calculations based solely on boluses (e.g., meal, correction, or other manually-initiated boluses). Additionally or alternatively, in some embodiments, based on the magnitude of the current residual active insulin, the infusion device may automatically generate or otherwise provide a graphical recommendation that the user consume carbohydrates, engage in (or disengage from) exercise or other physical activity, administer a correction bolus, or the like.

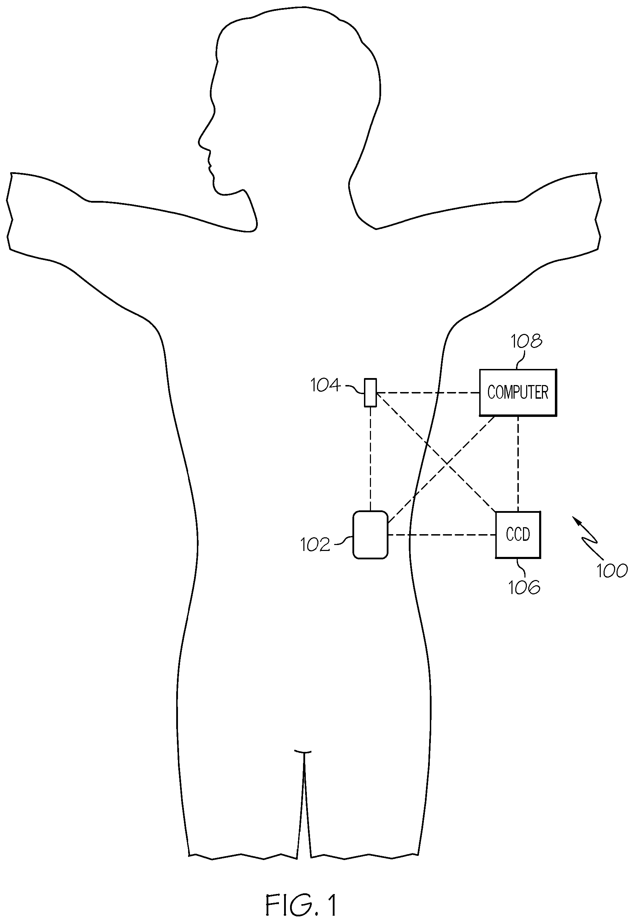

Turning now to FIG. 1, one exemplary embodiment of an infusion system 100 includes, without limitation, a fluid infusion device (or infusion pump) 102, a sensing arrangement 104, a command control device (CCD) 106, and a computer 108. The components of an infusion system 100 may be realized using different platforms, designs, and configurations, and the embodiment shown in FIG. 1 is not exhaustive or limiting. In practice, the infusion device 102 and the sensing arrangement 104 are secured at desired locations on the body of a user (or patient), as illustrated in FIG. 1. In this regard, the locations at which the infusion device 102 and the sensing arrangement 104 are secured to the body of the user in FIG. 1 are provided only as a representative, non-limiting, example. The elements of the infusion system 100 may be similar to those described in United States Patent No. 8,674,288.

In the illustrated embodiment of FIG. 1, the infusion device 102 is designed as a portable medical device suitable for infusing a fluid, a liquid, a gel, or other agent into the body of a user. In exemplary embodiments, the infused fluid is insulin, although many other fluids may be administered through infusion such as, but not limited to, HIV drugs, drugs to treat pulmonary hypertension, iron chelation drugs, pain medications, anti-cancer treatments, medications, vitamins, hormones, or the like. In some embodiments, the fluid may include a nutritional supplement, a dye, a tracing medium, a saline medium, a hydration medium, or the like.

The sensing arrangement 104 generally represents the components of the infusion system 100 configured to sense, detect, measure or otherwise quantify a condition of the user, and may include a sensor, a monitor, or the like, for providing data indicative of the condition that is sensed, detected, measured or otherwise monitored by the sensing arrangement. In this regard, the sensing arrangement 104 may include electronics and enzymes reactive to a biological condition, such as a blood glucose level, or the like, of the user, and provide data indicative of the blood glucose level to the infusion device 102, the CCD 106 and/or the computer 108. For example, the infusion device 102, the CCD 106 and/or the computer 108 may include a display for presenting information or data to the user based on the sensor data received from the sensing arrangement 104, such as, for example, a current glucose level of the user, a graph or chart of the user's glucose level versus time, device status indicators, alert messages, or the like. In other embodiments, the infusion device 102, the CCD 106 and/or the computer 108 may include electronics and software that are configured to analyze sensor data and operate the infusion device 102 to deliver fluid to the body of the user based on the sensor data and/or preprogrammed delivery routines. Thus, in exemplary embodiments, one or more of the infusion device 102, the sensing arrangement 104, the CCD 106, and/or the computer 108 includes a transmitter, a receiver, and/or other transceiver electronics that allow for communication with other components of the infusion system 100, so that the sensing arrangement 104 may transmit sensor data or monitor data to one or more of the infusion device 102, the CCD 106 and/or the computer 108.

Still referring to FIG. 1, in various embodiments, the sensing arrangement 104 may be secured to the body of the user or embedded in the body of the user at a location that is remote from the location at which the infusion device 102 is secured to the body of the user. In various other embodiments, the sensing arrangement 104 may be incorporated within the infusion device 102. In other embodiments, the sensing arrangement 104 may be separate and apart from the infusion device 102, and may be, for example, part of the CCD 106. In such embodiments, the sensing arrangement 104 may be configured to receive a biological sample, analyte, or the like, to measure a condition of the user.

As described above, in some embodiments, the CCD 106 and/or the computer 108 may include electronics and other components configured to perform processing, delivery routine storage, and to control the infusion device 102 in a manner that is influenced by sensor data measured by and/or received from the sensing arrangement 104. By including control functions in the CCD 106 and/or the computer 108, the infusion device 102 may be made with more simplified electronics. However, in other embodiments, the infusion device 102 may include all control functions, and may operate without the CCD 106 and/or the computer 108. In various embodiments, the CCD 106 may be a portable electronic device. In addition, in various embodiments, the infusion device 102 and/or the sensing arrangement 104 may be configured to transmit data to the CCD 106 and/or the computer 108 for display or processing of the data by the CCD 106 and/or the computer 108.

In some embodiments, the CCD 106 and/or the computer 108 may provide information to the user that facilitates the user's subsequent use of the infusion device 102. For example, the CCD 106 may provide information to the user to allow the user to determine the rate or dose of medication to be administered into the user's body. In other embodiments, the CCD 106 may provide information to the infusion device 102 to autonomously control the rate or dose of medication administered into the body of the user. In some embodiments, the sensing arrangement 104 may be integrated into the CCD 106. Such embodiments may allow the user to monitor a condition by providing, for example, a sample of his or her blood to the sensing arrangement 104 to assess his or her condition. In some embodiments, the sensing arrangement 104 and the CCD 106 may be used for determining glucose levels in the blood and/or body fluids of the user without the use of, or necessity of, a wire or cable connection between the infusion device 102 and the sensing arrangement 104 and/or the CCD 106.

In some embodiments, the sensing arrangement 104 and/or the infusion device 102 are cooperatively configured to utilize a closed-loop system for delivering fluid to the user. Examples of sensing devices and/or infusion pumps utilizing closed-loop systems may be found at, but are not limited to, the following United States patent numbers: 6,088,608, 6,119,028, 6,589,229, 6,740,072, 6,827,702, 7,323,142, and 7,402,153. In such embodiments, the sensing arrangement 104 is configured to sense or measure a condition of the user, such as, blood glucose level or the like. The infusion device 102 is configured to deliver fluid in response to the condition sensed by the sensing arrangement 104. In tum, the sensing arrangement 104 continues to sense or otherwise quantify a current condition of the user, thereby allowing the infusion device 102 to deliver fluid continuously in response to the condition currently (or most recently) sensed by the sensing arrangement 104 indefinitely. In some embodiments, the sensing arrangement 104 and/or the infusion device 102 may be configured to utilize the closed-loop system only for a portion of the day, for example only when the user is asleep or awake.

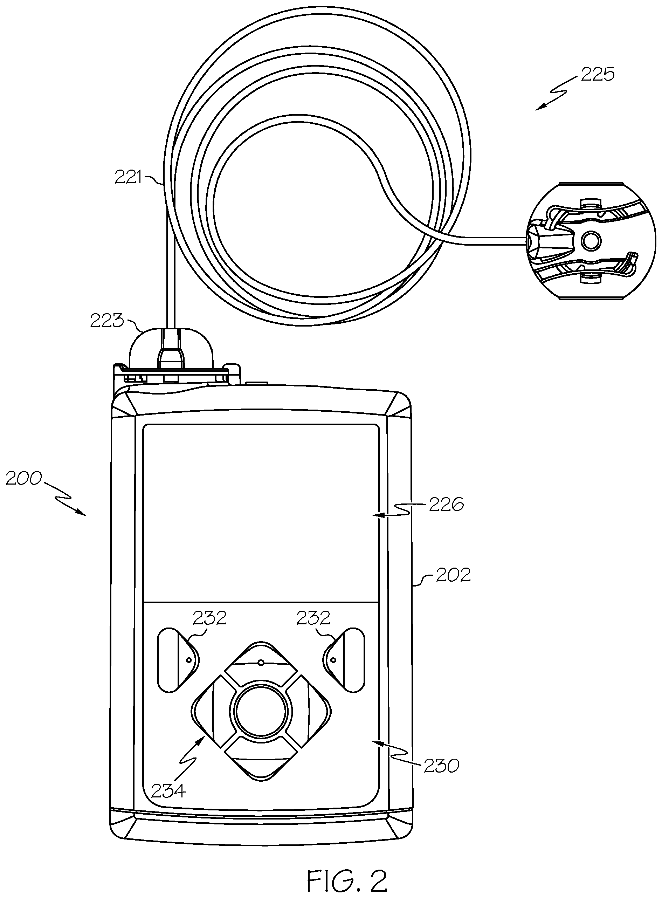

FIGS. 2-4 depict one exemplary embodiment of a fluid infusion device 200 (or alternatively, infusion pump) suitable for use in an infusion system, such as, for example, as infusion device 102 in the infusion system 100 of FIG. 1. The fluid infusion device 200 is a portable medical device designed to be carried or worn by a patient (or user), and the fluid infusion device 200 may leverage any number of conventional features, components, elements, and characteristics of existing fluid infusion devices, such as, for example, some of the features, components, elements, and/or characteristics described in United States Patent numbers 6,485,465 and 7,621,893. It should be appreciated that FIGS. 2-4 depict some aspects of the infusion device 200 in a simplified manner; in practice, the infusion device 200 could include additional elements, features, or components that are not shown or described in detail herein.

As best illustrated in FIGS. 2-3, the illustrated embodiment of the fluid infusion device 200 includes a housing 202 adapted to receive a fluid-containing reservoir 205. An opening 220 in the housing 202 accommodates a fitting 223 (or cap) for the reservoir 205, with the fitting 223 being configured to mate or otherwise interface with tubing 221 of an infusion set 225 that provides a fluid path to/from the body of the user. In this manner, fluid communication from the interior of the reservoir 205 to the user is established via the tubing 221. The illustrated fluid infusion device 200 includes a human-machine interface (HMI) 230 (or user interface) that includes elements 232, 234 that can be manipulated by the user to administer a bolus of fluid (e.g., insulin), to change therapy settings, to change user preferences, to select display features, and the like. The infusion device also includes a display element 226, such as a liquid crystal display (LCD) or another suitable display element, that can be used to present various types of information or data to the user, such as, without limitation: the current glucose level of the patient; the time; a graph or chart of the patient's glucose level versus time; device status indicators; etc.

The housing 202 is formed from a substantially rigid material having a hollow interior 214 adapted to allow an electronics assembly 204, a sliding member (or slide) 206, a drive system 208, a sensor assembly 210, and a drive system capping member 212 to be disposed therein in addition to the reservoir 205, with the contents of the housing 202 being enclosed by a housing capping member 216. The opening 220, the slide 206, and the drive system 208 are coaxially aligned in an axial direction (indicated by arrow 218), whereby the drive system 208 facilitates linear displacement of the slide 206 in the axial direction 218 to dispense fluid from the reservoir 205 (after the reservoir 205 has been inserted into opening 220), with the sensor assembly 210 being configured to measure axial forces (e.g., forces aligned with the axial direction 218) exerted on the sensor assembly 210 responsive to operating the drive system 208 to displace the slide 206. In various embodiments, the sensor assembly 210 may be utilized to detect one or more of the following: an occlusion in a fluid path that slows, prevents, or otherwise degrades fluid delivery from the reservoir 205 to a user's body; when the reservoir 205 is empty; when the slide 206 is properly seated with the reservoir 205; when a fluid dose has been delivered; when the infusion pump 200 is subjected to shock or vibration; when the infusion pump 200 requires maintenance.

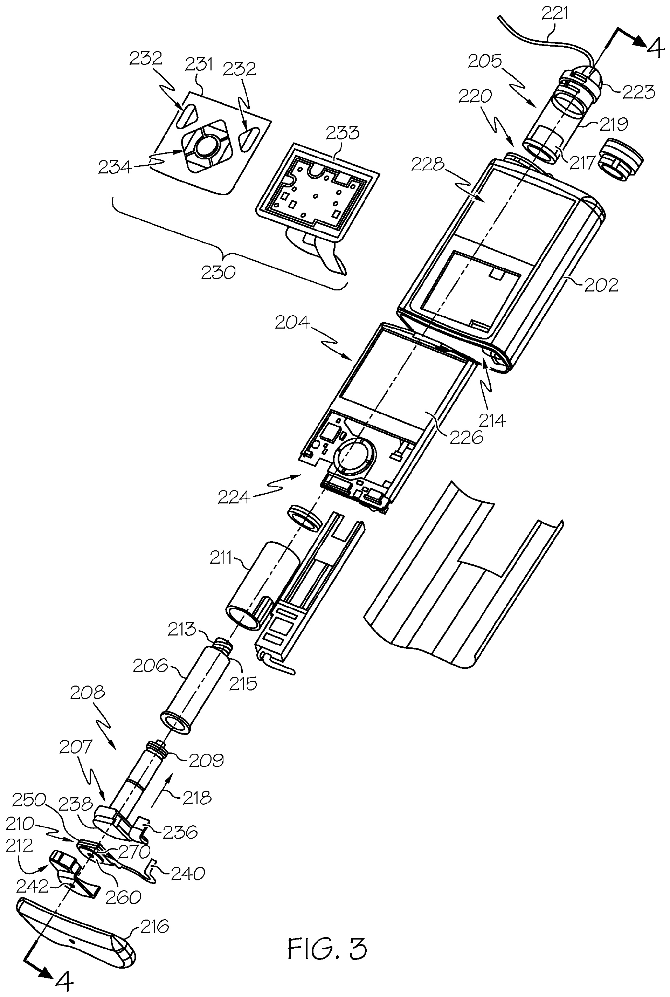

Depending on the embodiment, the fluid-containing reservoir 205 may be realized as a syringe, a vial, a cartridge, a bag, or the like. In certain embodiments, the infused fluid is insulin, although many other fluids may be administered through infusion such as, but not limited to, HIV drugs, drugs to treat pulmonary hypertension, iron chelation drugs, pain medications, anti-cancer treatments, medications, vitamins, hormones, or the like. As best illustrated in FIGS. 3-4, the reservoir 205 typically includes a reservoir barrel 219 that contains the fluid and is concentrically and/or coaxially aligned with the slide 206 (e.g., in the axial direction 218) when the reservoir 205 is inserted into the infusion pump 200. The end of the reservoir 205 proximate the opening 220 may include or otherwise mate with the fitting 223, which secures the reservoir 205 in the housing 202 and prevents displacement of the reservoir 205 in the axial direction 218 with respect to the housing 202 after the reservoir 205 is inserted into the housing 202. As described above, the fitting 223 extends from (or through) the opening 220 of the housing 202 and mates with tubing 221 to establish fluid communication from the interior of the reservoir 205 (e.g., reservoir barrel 219) to the user via the tubing 221 and infusion set 225. The opposing end of the reservoir 205 proximate the slide 206 includes a plunger 217 (or stopper) positioned to push fluid from inside the barrel 219 of the reservoir 205 along a fluid path through tubing 221 to a user. The slide 206 is configured to mechanically couple or otherwise engage with the plunger 217, thereby becoming seated with the plunger 217 and/or reservoir 205. Fluid is forced from the reservoir 205 via tubing 221 as the drive system 208 is operated to displace the slide 206 in the axial direction 218 toward the opening 220 in the housing 202.

In the illustrated embodiment of FIGS. 3-4, the drive system 208 includes a motor assembly 207 and a drive screw 209. The motor assembly 207 includes a motor that is coupled to drive train components of the drive system 208 that are configured to convert rotational motor motion to a translational displacement of the slide 206 in the axial direction 218, and thereby engaging and displacing the plunger 217 of the reservoir 205 in the axial direction 218. In some embodiments, the motor assembly 207 may also be powered to translate the slide 206 in the opposing direction (e.g., the direction opposite direction 218) to retract and/or detach from the reservoir 205 to allow the reservoir 205 to be replaced. In exemplary embodiments, the motor assembly 207 includes a brushless DC (BLDC) motor having one or more permanent magnets mounted, affixed, or otherwise disposed on its rotor. However, the subject matter described herein is not necessarily limited to use with BLDC motors, and in alternative embodiments, the motor may be realized as a solenoid motor, an AC motor, a stepper motor, a piezoelectric caterpillar drive, a shape memory actuator drive, an electrochemical gas cell, a thermally driven gas cell, a bimetallic actuator, or the like. The drive train components may comprise one or more lead screws, cams, ratchets, jacks, pulleys, pawls, clamps, gears, nuts, slides, bearings, levers, beams, stoppers, plungers, sliders, brackets, guides, bearings, supports, bellows, caps, diaphragms, bags, heaters, or the like. In this regard, although the illustrated embodiment of the infusion pump utilizes a coaxially aligned drive train, the motor could be arranged in an offset or otherwise non-coaxial manner, relative to the longitudinal axis of the reservoir 205.

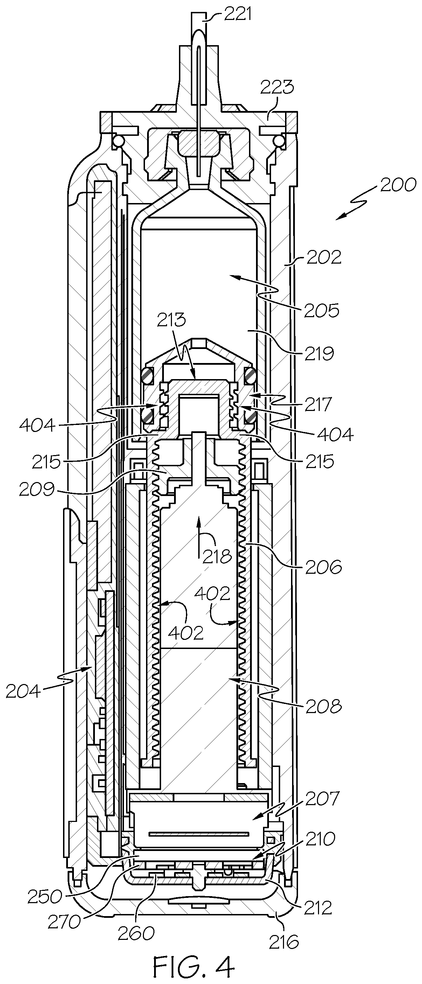

As best shown in FIG. 4, the drive screw 209 mates with threads 402 internal to the slide 206. When the motor assembly 207 is powered and operated, the drive screw 209 rotates, and the slide 206 is forced to translate in the axial direction 218. In an exemplary embodiment, the infusion pump 200 includes a sleeve 211 to prevent the slide 206 from rotating when the drive screw 209 of the drive system 208 rotates. Thus, rotation of the drive screw 209 causes the slide 206 to extend or retract relative to the drive motor assembly 207. When the fluid infusion device is assembled and operational, the slide 206 contacts the plunger 217 to engage the reservoir 205 and control delivery of fluid from the infusion pump 200. In an exemplary embodiment, the shoulder portion 215 of the slide 206 contacts or otherwise engages the plunger 217 to displace the plunger 217 in the axial direction 218. In alternative embodiments, the slide 206 may include a threaded tip 213 capable of being detachably engaged with internal threads 404 on the plunger 217 of the reservoir 205, as described in detail in United States patent numbers 6,248,093 and 6,485,465.

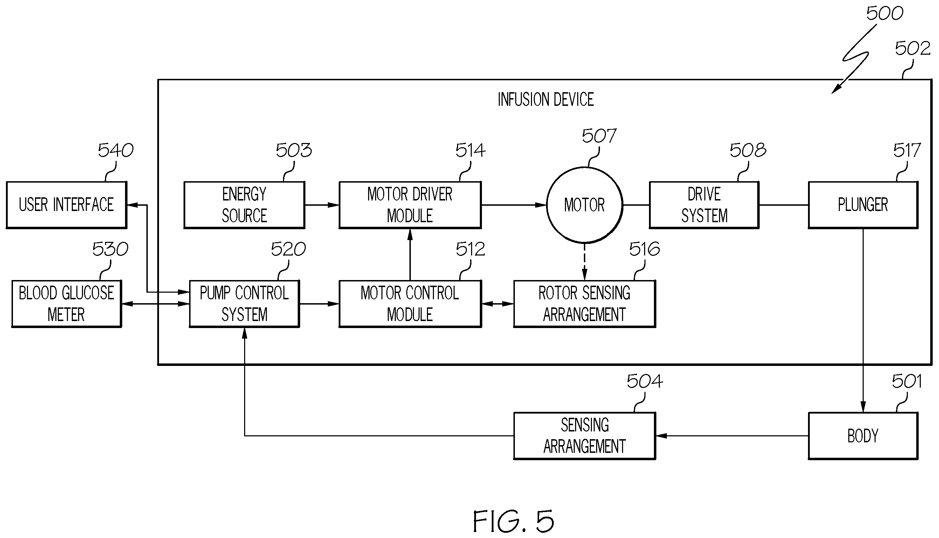

As illustrated in FIG. 3, the electronics assembly 204 includes control electronics 224 coupled to the display element 226, with the housing 202 including a transparent window portion 228 that is aligned with the display element 226 to allow the display 226 to be viewed by the user when the electronics assembly 204 is disposed within the interior 214 of the housing 202. The control electronics 224 generally represent the hardware, firmware, processing logic and/or software (or combinations thereof) configured to control operation of the motor assembly 207 and/or drive system 208, as described in greater detail below in the context of FIG. 5. Whether such functionality is implemented as hardware, firmware, a state machine, or software depends upon the particular application and design constraints imposed on the embodiment. Those familiar with the concepts described here may implement such functionality in a suitable manner for each particular application, but such implementation decisions should not be interpreted as being restrictive or limiting. In an exemplary embodiment, the control electronics 224 includes one or more programmable controllers that may be programmed to control operation of the infusion pump 200.

The motor assembly 207 includes one or more electrical leads 236 adapted to be electrically coupled to the electronics assembly 204 to establish communication between the control electronics 224 and the motor assembly 207. In response to command signals from the control electronics 224 that operate a motor driver (e.g., a power converter) to regulate the amount of power supplied to the motor from a power supply, the motor actuates the drive train components of the drive system 208 to displace the slide 206 in the axial direction 218 to force fluid from the reservoir 205 along a fluid path (including tubing 221 and an infusion set), thereby administering doses of the fluid contained in the reservoir 205 into the user's body. Preferably, the power supply is realized one or more batteries contained within the housing 202. Alternatively, the power supply may be a solar panel, capacitor, AC or DC power supplied through a power cord, or the like. In some embodiments, the control electronics 224 may operate the motor of the motor assembly 207 and/or drive system 208 in a stepwise manner, typically on an intermittent basis; to administer discrete precise doses of the fluid to the user according to programmed delivery profiles.

Referring to FIGS. 2-4, as described above, the user interface 230 includes HMI elements, such as buttons 232 and a directional pad 234, that are formed on a graphic keypad overlay 231 that overlies a keypad assembly 233, which includes features corresponding to the buttons 232, directional pad 234 or other user interface items indicated by the graphic keypad overlay 231. When assembled, the keypad assembly 233 is coupled to the control electronics 224, thereby allowing the HMI elements 232, 234 to be manipulated by the user to interact with the control electronics 224 and control operation of the infusion pump 200, for example, to administer a bolus of insulin, to change therapy settings, to change user preferences, to select display features, to set or disable alarms and reminders, and the like. In this regard, the control electronics 224 maintains and/or provides information to the display 226 regarding program parameters, delivery profiles, pump operation, alarms, warnings, statuses, or the like, which may be adjusted using the HMI elements 232, 234. In various embodiments, the HMI elements 232, 234 may be realized as physical objects (e.g., buttons, knobs, joysticks, and the like) or virtual objects (e.g., using touch-sensing and/or proximity-sensing technologies). For example, in some embodiments, the display 226 may be realized as a touch screen or touch-sensitive display, and in such embodiments, the features and/or functionality of the HMI elements 232, 234 may be integrated into the display 226 and the HMI 230 may not be present. In some embodiments, the electronics assembly 204 may also include alert generating elements coupled to the control electronics 224 and suitably configured to generate one or more types of feedback, such as, without limitation: audible feedback; visual feedback; haptic (physical) feedback; or the like.

Referring to FIGS. 3-4, in accordance with one or more embodiments, the sensor assembly 210 includes a back plate structure 250 and a loading element 260. The loading element 260 is disposed between the capping member 212 and a beam structure 270 that includes one or more beams having sensing elements disposed thereon that are influenced by compressive force applied to the sensor assembly 210 that deflects the one or more beams, as described in greater detail in United States Patent No. 8,474,332. In exemplary embodiments, the back plate structure 250 is affixed, adhered, mounted, or otherwise mechanically coupled to the bottom surface 238 of the drive system 208 such that the back plate structure 250 resides between the bottom surface 238 of the drive system 208 and the housing cap 216. The drive system capping member 212 is contoured to accommodate and conform to the bottom of the sensor assembly 210 and the drive system 208. The drive system capping member 212 may be affixed to the interior of the housing 202 to prevent displacement of the sensor assembly 210 in the direction opposite the direction of force provided by the drive system 208 (e.g., the direction opposite direction 218). Thus, the sensor assembly 210 is positioned between the motor assembly 207 and secured by the capping member 212, which prevents displacement of the sensor assembly 210 in a downward direction opposite the direction of arrow 218, such that the sensor assembly 210 is subjected to a reactionary compressive force when the drive system 208 and/or motor assembly 207 is operated to displace the slide 206 in the axial direction 218 in opposition to the fluid pressure in the reservoir 205. Under normal operating conditions, the compressive force applied to the sensor assembly 210 is correlated with the fluid pressure in the reservoir 205. As shown, electrical leads 240 are adapted to electrically couple the sensing elements of the sensor assembly 210 to the electronics assembly 204 to establish communication to the control electronics 224, wherein the control electronics 224 are configured to measure, receive, or otherwise obtain electrical signals from the sensing elements of the sensor assembly 210 that are indicative of the force applied by the drive system 208 in the axial direction 218.

FIG. 5 depicts an exemplary embodiment of a control system 500 suitable for use with an infusion device 502, such as the infusion device 102 in FIG. 1 or the infusion device 200 of FIG. 2. The control system 500 is capable of controlling or otherwise regulating a physiological condition in the body 501 of a user to a desired (or target) value or otherwise maintain the condition within a range of acceptable values in an automated manner. In one or more exemplary embodiments, the condition being regulated is sensed, detected, measured or otherwise quantified by a sensing arrangement 504 (e.g., sensing arrangement 104) communicatively coupled to the infusion device 502. However, it should be noted that in alternative embodiments, the condition being regulated by the control system 500 may be correlative to the measured values obtained by the sensing arrangement 504. That said, for clarity and purposes of explanation, the subject matter may be described herein in the context of the sensing arrangement 504 being realized as a glucose sensing arrangement that senses, detects, measures or otherwise quantifies the user's glucose level, which is being regulated in the body 501 of the user by the control system 500.

In exemplary embodiments, the sensing arrangement 504 includes one or more interstitial glucose sensing elements that generate or otherwise output electrical signals having a signal characteristic that is correlative to, influenced by, or otherwise indicative of the relative interstitial fluid glucose level in the body 501 of the user. The output electrical signals are filtered or otherwise processed to obtain a measurement value indicative of the user's interstitial fluid glucose level. In exemplary embodiments, a blood glucose meter 530, such as a finger stick device, is utilized to directly sense, detect, measure or otherwise quantify the blood glucose in the body 501 of the user. In this regard, the blood glucose meter 530 outputs or otherwise provides a measured blood glucose value that may be utilized as a reference measurement for calibrating the sensing arrangement 504 and converting a measurement value indicative of the user's interstitial fluid glucose level into a corresponding calibrated blood glucose value. For purposes of explanation, the calibrated blood glucose value calculated based on the electrical signals output by the sensing element(s) of the sensing arrangement 504 may alternatively be referred to herein as the sensor glucose value, the sensed glucose value, or variants thereof.

In the illustrated embodiment, the pump control system 520 generally represents the electronics and other components of the infusion device 502 that control operation of the fluid infusion device 502 according to a desired infusion delivery program in a manner that is influenced by the sensed glucose value indicative of a current glucose level in the body 501 of the user. For example, to support a closed-loop operating mode, the pump control system 520 maintains, receives, or otherwise obtains a target or commanded glucose value, and automatically generates or otherwise determines dosage commands for operating the motor 507 to displace the plunger 517 and deliver insulin to the body 501 of the user based on the difference between a sensed glucose value and the target glucose value. In other operating modes, the pump control system 520 may generate or otherwise determine dosage commands configured to maintain the sensed glucose value below an upper glucose limit, above a lower glucose limit, or otherwise within a desired range of glucose values. In practice, the infusion device 502 may store or otherwise maintain the target value, upper and/or lower glucose limit(s), and/or other glucose threshold value(s) in a data storage element accessible to the pump control system 520.

The target glucose value and other threshold glucose values may be received from an external component (e.g., CCD 106 and/or computing device 108) or be input by a user via a user interface element 540 associated with the infusion device 502. In practice, the one or more user interface element(s) 540 associated with the infusion device 502 typically include at least one input user interface element, such as, for example, a button, a keypad, a keyboard, a knob, a joystick, a mouse, a touch panel, a touchscreen, a microphone or another audio input device, and/or the like. Additionally, the one or more user interface element(s) 540 include at least one output user interface element, such as, for example, a display element (e.g., a light-emitting diode or the like), a display device (e.g., a liquid crystal display or the like), a speaker or another audio output device, a haptic feedback device, or the like, for providing notifications or other information to the user. It should be noted that although FIG. 5 depicts the user interface element(s) 540 as being separate from the infusion device 502, in practice, one or more of the user interface element(s) 540 may be integrated with the infusion device 502. Furthermore, in some embodiments, one or more user interface element(s) 540 are integrated with the sensing arrangement 504 in addition to and/or in alternative to the user interface element(s) 540 integrated with the infusion device 502. The user interface element(s) 540 may be manipulated by the user to operate the infusion device 502 to deliver correction boluses, adjust target and/or threshold values, modify the delivery control scheme or operating mode, and the like, as desired.

Still referring to FIG. 5, in the illustrated embodiment, the infusion device 502 includes a motor control module 512 coupled to a motor 507 (e.g., motor assembly 207) that is operable to displace a plunger 517 (e.g., plunger 217) in a reservoir (e.g., reservoir 205) and provide a desired amount of fluid to the body 501 of a user. In this regard, displacement of the plunger 517 results in the delivery of a fluid that is capable of influencing the condition in the body 501 of the user to the body 501 of the user via a fluid delivery path (e.g., via tubing 221 of an infusion set 225). A motor driver module 514 is coupled between an energy source 503 and the motor 507. The motor control module 512 is coupled to the motor driver module 514, and the motor control module 512 generates or otherwise provides command signals that operate the motor driver module 514 to provide current (or power) from the energy source 503 to the motor 507 to displace the plunger 517 in response to receiving, from a pump control system 520, a dosage command indicative of the desired amount of fluid to be delivered.

In exemplary embodiments, the energy source 503 is realized as a battery housed within the infusion device 502 (e.g., within housing 202) that provides direct current (DC) power. In this regard, the motor driver module 514 generally represents the combination of circuitry, hardware and/or other electrical components configured to convert or otherwise transfer DC power provided by the energy source 503 into alternating electrical signals applied to respective phases of the stator windings of the motor 507 that result in current flowing through the stator windings that generates a stator magnetic field and causes the rotor of the motor 507 to rotate. The motor control module 512 is configured to receive or otherwise obtain a commanded dosage from the pump control system 520, convert the commanded dosage to a commanded translational displacement of the plunger 517, and command, signal, or otherwise operate the motor driver module 514 to cause the rotor of the motor 507 to rotate by an amount that produces the commanded translational displacement of the plunger 517. For example, the motor control module 512 may determine an amount of rotation of the rotor required to produce translational displacement of the plunger 517 that achieves the commanded dosage received from the pump control system 520. Based on the current rotational position (or orientation) of the rotor with respect to the stator that is indicated by the output of the rotor sensing arrangement 516, the motor control module 512 determines the appropriate sequence of alternating electrical signals to be applied to the respective phases of the stator windings that should rotate the rotor by the determined amount of rotation from its current position (or orientation). In embodiments where the motor 507 is realized as a BLDC motor, the alternating electrical signals commutate the respective phases of the stator windings at the appropriate orientation of the rotor magnetic poles with respect to the stator and in the appropriate order to provide a rotating stator magnetic field that rotates the rotor in the desired direction. Thereafter, the motor control module 512 operates the motor driver module 514 to apply the determined alternating electrical signals (e.g., the command signals) to the stator windings of the motor 507 to achieve the desired delivery of fluid to the user.

When the motor control module 512 is operating the motor driver module 514, current flows from the energy source 503 through the stator windings of the motor 507 to produce a stator magnetic field that interacts with the rotor magnetic field. In some embodiments, after the motor control module 512 operates the motor driver module 514 and/or motor 507 to achieve the commanded dosage, the motor control module 512 ceases operating the motor driver module 514 and/or motor 507 until a subsequent dosage command is received. In this regard, the motor driver module 514 and the motor 507 enter an idle state during which the motor driver module 514 effectively disconnects or isolates the stator windings of the motor 507 from the energy source 503. In other words, current does not flow from the energy source 503 through the stator windings of the motor 507 when the motor 507 is idle, and thus, the motor 507 does not consume power from the energy source 503 in the idle state, thereby improving efficiency.

Depending on the embodiment, the motor control module 512 may be implemented or realized with a general purpose processor, a microprocessor, a controller, a microcontroller, a state machine, a content addressable memory, an application specific integrated circuit, a field programmable gate array, any suitable programmable logic device, discrete gate or transistor logic, discrete hardware components, or any combination thereof, designed to perform the functions described herein. In exemplary embodiments, the motor control module 512 includes or otherwise accesses a data storage element or memory, including any sort of random access memory (RAM), read only memory (ROM), flash memory, registers, hard disks, removable disks, magnetic or optical mass storage, or any other short or long term storage media or other non-transitory computer-readable medium, which is capable of storing programming instructions for execution by the motor control module 512. The computer-executable programming instructions, when read and executed by the motor control module 512, cause the motor control module 512 to perform or otherwise support the tasks, operations, functions, and processes described herein.

It should be appreciated that FIG. 5 is a simplified representation of the infusion device 502 for purposes of explanation and is not intended to limit the subject matter described herein in any way. In this regard, depending on the embodiment, some features and/or functionality of the sensing arrangement 504 may implemented by or otherwise integrated into the pump control system 520, or vice versa. Similarly, in practice, the features and/or functionality of the motor control module 512 may implemented by or otherwise integrated into the pump control system 520, or vice versa. Furthermore, the features and/or functionality of the pump control system 520 may be implemented by control electronics 224 located in the fluid infusion device 200, 400, while in alternative embodiments, the pump control system 520 may be implemented by a remote computing device that is physically distinct and/or separate from the infusion device 502, such as, for example, the CCD 106 or the computing device 108.

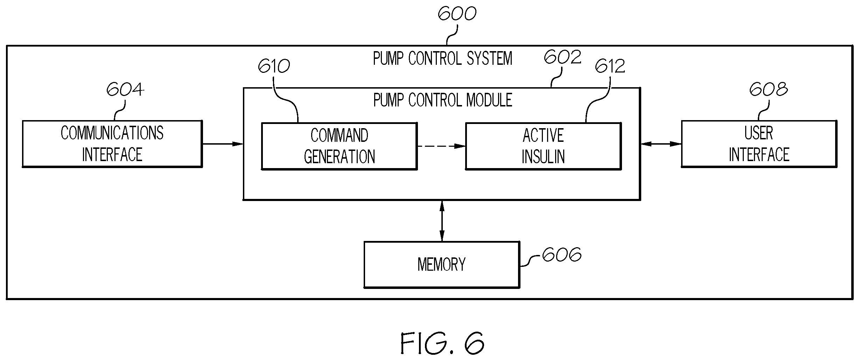

FIG. 6 depicts an exemplary embodiment of a pump control system 600 suitable for use as the pump control system 520 in FIG. 5 in accordance with one or more embodiments. The illustrated pump control system 600 includes, without limitation, a pump control module 602, a communications interface 604, and a data storage element (or memory) 606. The pump control module 602 is coupled to the communications interface 604 and the memory 606, and the pump control module 602 is suitably configured to support the operations, tasks, and/or processes described herein. In exemplary embodiments, the pump control module 602 is also coupled to one or more user interface elements 608 (e.g., user interface 230, 540) for receiving user input and providing notifications, alerts, or other therapy information to the user. Although FIG. 6 depicts the user interface element 608 as being integrated with the pump control system 600 (e.g., as part of the infusion device 200, 502), in various alternative embodiments, the user interface element 608 may be integrated with the sensing arrangement 504 or another element of an infusion system 100 (e.g., the computer 108 or CCD 106).

Referring to FIG. 6 and with reference to FIG. 5, the communications interface 604 generally represents the hardware, circuitry, logic, firmware and/or other components of the pump control system 600 that are coupled to the pump control module 602 and configured to support communications between the pump control system 600 and the sensing arrangement 504. In this regard, the communications interface 604 may include or otherwise be coupled to one or more transceiver modules capable of supporting wireless communications between the pump control system 520, 600 and the sensing arrangement 504 or another electronic device 106, 108 in an infusion system 100. In other embodiments, the communications interface 604 may be configured to support wired communications to/from the sensing arrangement 504.

The pump control module 602 generally represents the hardware, circuitry, logic, firmware and/or other component of the pump control system 600 that is coupled to the communications interface 604 and configured to determine dosage commands for operating the motor 506 to deliver fluid to the body 501 based on data received from the sensing arrangement 504 and perform various additional tasks, operations, functions and/or operations described herein. For example, in exemplary embodiments, pump control module 602 implements or otherwise executes a command generation application 610 that supports one or more autonomous operating modes and calculates or otherwise determines dosage commands for operating the motor 506 of the infusion device 502 in an autonomous operating mode based at least in part on a current measurement value for a condition in the body 501 of the user. For example, in a closed-loop operating mode, the command generation application 610 may determine a dosage command for operating the motor 506 to deliver insulin to the body 501 of the user based at least in part on the current glucose measurement value most recently received from the sensing arrangement 504 to regulate the user's blood glucose level to a target reference glucose value. Additionally, the command generation application 610 may generate dosage commands for boluses that are manually-initiated or otherwise instructed by a user via a user interface element 608. For example, regardless of the operating mode being implemented, the command generation application 610 may determine a dosage command for operating the motor 506 to deliver a bolus insulin to the body 501 of the user corresponding to a correction bolus amount selected or otherwise indicated by the user via the user interface element 230, 540, 608.

In exemplary embodiments, pump control module 602 also implements or otherwise executes an active insulin application 612 that calculates or otherwise determines one or more active insulin metrics based on the dosage commands generated by the command generation application 610 and generates or otherwise provides user notifications or alerts via a user interface element 608 based at least in part on a current value for an active insulin metric. As described in greater detail below in the context of FIG. 8, in exemplary embodiments, the active insulin application 612 calculates or otherwise determines values for a residual amount of insulin active in the body 501 of the user based on the variable basal rate dosage commands generated by the command generation application 610 in an autonomous operating mode and automatically generates one or more user notifications in a manner that is influenced by the value of the residual insulin metric when exiting or otherwise transitioning from the autonomous operating mode.

Still referring to FIG. 6, depending on the embodiment, the pump control module 602 may be implemented or realized with a general purpose processor, a microprocessor, a controller, a microcontroller, a state machine, a content addressable memory, an application specific integrated circuit, a field programmable gate array, any suitable programmable logic device, discrete gate or transistor logic, discrete hardware components, or any combination thereof, designed to perform the functions described herein. In this regard, the steps of a method or algorithm described in connection with the embodiments disclosed herein may be embodied directly in hardware, in firmware, in a software module executed by the pump control module 602, or in any practical combination thereof. In exemplary embodiments, the pump control module 602 includes or otherwise accesses the data storage element or memory 606, which may be realized using any sort of non-transitory computer-readable medium capable of storing programming instructions for execution by the pump control module 602. The computer-executable programming instructions, when read and executed by the pump control module 602, cause the pump control module 602 to implement or otherwise generate one or more of the applications 612, 610 and perform the tasks, operations, functions, and processes described in greater detail below.

It should be understood that FIG. 6 is a simplified representation of a pump control system 600 for purposes of explanation and is not intended to limit the subject matter described herein in any way. For example, in some embodiments, the features and/or functionality of the motor control module 512 may be implemented by or otherwise integrated into the pump control system 600 and/or the pump control module 602, for example, by the command generation application 610 converting the dosage command into a corresponding motor command, in which case, the separate motor control module 512 may be absent from an embodiment of the infusion device 502.

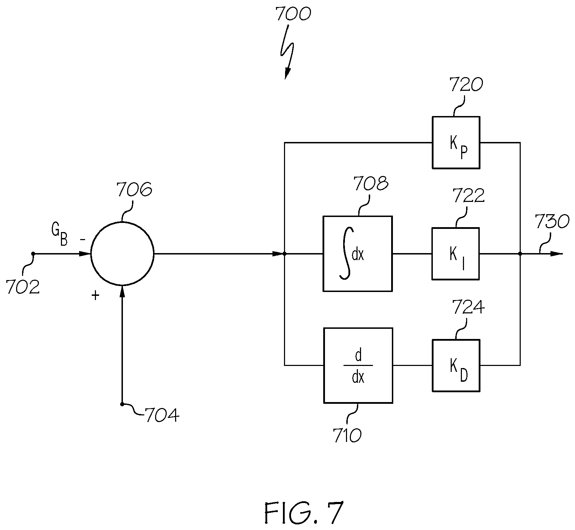

FIG. 7 depicts an exemplary closed-loop control system 700 that may be implemented by a pump control system 520, 600 to provide a closed-loop operating mode that autonomously regulates a condition in the body of a user to a reference (or target) value. It should be appreciated that FIG. 7 is a simplified representation of the control system 700 for purposes of explanation and is not intended to limit the subject matter described herein in any way.

In exemplary embodiments, the control system 700 receives or otherwise obtains a target glucose value at input 702. In some embodiments, the target glucose value may be stored or otherwise maintained by the infusion device 502 (e.g., in memory 606), however, in some alternative embodiments, the target value may be received from an external component (e.g., CCD 106 and/or computer 108). In one or more embodiments, the target glucose value may be dynamically calculated or otherwise determined prior to entering the closed-loop operating mode based on one or more patient-specific control parameters. For example, the target blood glucose value may be calculated based at least in part on a patient-specific reference basal rate and a patient-specific daily insulin requirement, which are determined based on historical delivery information over a preceding interval of time (e.g., the amount of insulin delivered over the preceding 24 hours). The control system 700 also receives or otherwise obtains a current glucose measurement value from the sensing arrangement 504 at input 704. The illustrated control system 700 implements or otherwise provides proportional-integral-derivative (PID) control to determine or otherwise generate delivery commands for operating the motor 510 based at least in part on the difference between the target glucose value and the current glucose measurement value. In this regard, the PID control attempts to minimize the difference between the measured value and the target value, and thereby regulates the measured value to the desired value. PID control parameters are applied to the difference between the target glucose level at input 702 and the measured glucose level at input 704 to generate or otherwise determine a dosage (or delivery) command provided at output 730. Based on that delivery command, the motor control module 512 operates the motor 510 to deliver insulin to the body of the user to influence the user's glucose level, and thereby reduce the difference between a subsequently measured glucose level and the target glucose level.

The illustrated control system 700 includes or otherwise implements a summation block 706 configured to determine a difference between the target value obtained at input 702 and the measured value obtained from the sensing arrangement 504 at input 704, for example, by subtracting the target value from the measured value. The output of the summation block 706 represents the difference between the measured and target values, which is then provided to each of a proportional term path, an integral term path, and a derivative term path. The proportional term path includes a gain block 720 that multiplies the difference by a proportional gain coefficient, $K_P$, to obtain the proportional term. The integral term path includes an integration block 708 that integrates the difference and a gain block 722 that multiplies the integrated difference by an integral gain coefficient, $K_I$, to obtain the integral term. The derivative term path includes a derivative block 710 that determines the derivative of the difference and a gain block 724 that multiplies the derivative of the difference by a derivative gain coefficient, $K_D$, to obtain the derivative term. The proportional term, the integral term, and the derivative term are then added or otherwise combined to obtain a delivery command that is utilized to operate the motor at output 730. Various implementation details pertaining to closed-loop PID control and determine gain coefficients are described in greater detail in United States patent number 7,402,153.

In one or more exemplary embodiments, the PID gain coefficients are user-specific (or patient-specific) and dynamically calculated or otherwise determined prior to entering the closed-loop operating mode based on historical insulin delivery information (e.g., amounts and/or timings of previous dosages, historical correction bolus information, or the like), historical sensor measurement values, historical reference blood glucose measurement values, user-reported or user-input events (e.g., meals, exercise, and the like), and the like. In this regard, one or more patient-specific control parameters (e.g., an insulin sensitivity factor, a daily insulin requirement, an insulin limit, a reference basal rate, a reference fasting glucose, an active insulin action duration, pharmodynamical time constants, or the like) may be utilized to compensate, correct, or otherwise adjust the PID gain coefficients to account for various operating conditions experienced and/or exhibited by the infusion device 502. The PID gain coefficients may be maintained by the memory 606 accessible to the pump control module 602. In this regard, the memory 606 may include a plurality of registers associated with the control parameters for the PID control. For example, a first parameter register may store the target glucose value and be accessed by or otherwise coupled to the summation block 706 at input 702, and similarly, a second parameter register accessed by the proportional gain block 720 may store the proportional gain coefficient, a third parameter register accessed by the integration gain block 722 may store the integration gain coefficient, and a fourth parameter register accessed by the derivative gain block 724 may store the derivative gain coefficient.

FIG. 8 depicts an exemplary active insulin notification process 800 suitable for implementation by a control system associated with a fluid infusion device, such as the control system 500 in the infusion device 502, to notify the user of the current status of the insulin in the body of the user when transitioning from one operating mode to another operating mode. For purposes of explanation, the subject matter is described herein in the context of providing notifications when transitioning from a closed-loop operating mode to an open-loop operating mode; however, it should be appreciated that the subject matter described herein is not limited to any particular initial operating mode or destination operating mode.

It is also noted that while the description herein discusses the active fluid as insulin used for diabetes control, the same structures and method can be used for the control of other physiological conditions using an appropriate other active fluid. In other words, in practice, the active insulin notification process may be generalized to an active fluid notification process for generating notifications in a similar manner as described herein based on similar amounts of a different type of active fluid in an equivalent manner.

The various tasks performed in connection with the active insulin notification process 800 may be performed by hardware, firmware, software executed by processing circuitry, or any combination thereof. For illustrative purposes, the following description refers to elements mentioned above in connection with FIGS. 1-7. In practice, portions of the active insulin notification process 800 may be performed by different elements of the control system 500, such as, for example, the infusion device 502, the sensing arrangement 504, the pump control system 520, 600, the pump control module 602, the active insulin application 612, the command generation application 610, and/or the user interface 540, 608. It should be appreciated that the active insulin notification process 800 may include any number of additional or alternative tasks, the tasks need not be performed in the illustrated order and/or the tasks may be performed concurrently, and/or the active insulin notification process 800 may be incorporated into a more comprehensive procedure or process having additional functionality not described in detail herein. Moreover, one or more of the tasks shown and described in the context of FIG. 8 could be omitted from a practical embodiment of the active insulin notification process 800 as long as the intended overall functionality remains intact.

Referring to FIG. 8 with continued reference to FIGS. 5-7, in exemplary embodiments, the active insulin notification process 800 initializes or otherwise begins by calculating or otherwise determining an initial amount of insulin that is active in the body of the patient upon entering the closed-loop operating mode (task 802). For example, when transitioning from an open-loop operating mode, the pump control system 520, 600 may calculate or otherwise determine an initial insulin-on-board for the patient based on the manually-initiated boluses delivered to the patient while in the open-loop mode. In this regard, based on the respective amounts of insulin delivered for the various meal or correction boluses administered by the patient, the respective timing of those boluses, and various absorption time constants, the pump control system 520, 600 and/or pump control module 602 may determine the current amount of active insulin in the body 501 of the patient for use as the initial active insulin amount upon entry to the closed-loop operating mode.

In exemplary embodiments described herein, initial active insulin amounts are determined or otherwise obtained for pharmacokinetics compartments used to model the patient's metabolization of insulin, namely, the subcutaneous, plasma, and effect-site compartments. In this regard, for the delivery of a fluid, such as insulin or another drug, through subcutaneous infusion or injection, the infused fluid has to be absorbed from the tissue into the blood circulation and then diffuse from the blood into other tissues where the infused fluid is to have its effect. Accordingly, in the context of insulin infusion, the subcutaneous compartment corresponds to the tissue space at which the insulin is infused, the plasma compartment corresponds to the blood in circulation, and the effect-site compartment corresponds to the tissues where insulin has an effect (e.g., the liver and muscle, among other tissues). In other words, the amount of active insulin in the subcutaneous compartment is the amount of active insulin in the subcutaneous tissue yet to be absorbed into the circulatory system, the amount of active insulin in the plasma compartment is the amount of active insulin in the blood plasma yet to diffuse to the tissues where insulin has an effect, and the amount of active insulin in the effect-site compartment is the amount of active insulin that has diffused to but not yet metabolized by those effect-site tissues. In one or more embodiments, the pump control system 520, 600 and/or pump control module 602 utilizes a lookup table to identify the current amount of active insulin in the pharmacokinetics compartments in the open-loop operating mode.

Referring again to FIG. 8, the illustrated process 800 continues by receiving or otherwise obtaining a current glucose measurement value for the patient and autonomously operating the infusion device in the closed-loop operating mode based on the current glucose measurement value (tasks 804, 806). As described above, the pump control system 520, 600 and/or pump control module 602 receives or otherwise obtains a glucose measurement value from the sensing arrangement 504, and based on a difference between the glucose measurement value and a reference glucose measurement value, the command generation application 610 generates or otherwise provides a dosage command corresponding to an amount of insulin to be delivered to reduce the difference between the glucose measurement value and the reference glucose measurement value, as described above in the context of FIG. 7. In this regard, as the difference between the most recent glucose measurement value and the reference glucose measurement value varies, the dosage amount determined by the command generation application 610 will vary in a corresponding manner, thereby effectuating a variable basal rate of insulin infusion while in the closed-loop operating mode. The variable basal rate dosage commands determined by the command generation application 610 are converted into corresponding motor commands, which, in turn, are utilized by the motor control module 512 to operate the motor 507 and deliver insulin at the variable basal rate. In this manner, in the closed-loop operating mode, the pump control system 520, 600 and/or pump control module 602 autonomously operates the motor 507 of the infusion device 502 to deliver a variable basal rate of infusion and regulate the patient's current glucose measurement value to the patient's target glucose value. That said, it should be noted that while in the closed-loop operating mode, the patient may still interact with the infusion device 502 to manually administer a correction bolus as desired. However, depending on the duration of the closed-loop operations and the magnitude of the correction boluses, the total amount of insulin delivered autonomously via the variable basal rate determined by the closed-loop control system 700 may be greater than the amount of insulin delivered via manually-initiated correction boluses.

In exemplary embodiments, the active insulin notification process 800 obtains closed-loop delivery data including the variable basal rate dosages autonomously determined by the closed-loop control system and recursively calculating or otherwise determining a total amount of active insulin in the body of the patient based on the closed-loop delivery data (tasks 808, 810). The active insulin application 612 may receive or otherwise obtain the dosage commands (or the corresponding dosage amounts) from the command generation application 610 and recursively calculate the current total amount of active insulin in the body of the patient based on the current dosage command and one or more preceding amounts of active insulin for each iteration of the loop defined by tasks 804, 806, 808, 810, 812 and 814 of the active insulin notification process 800. For example, the active insulin application 612 may utilize the initial dosage command obtained from the command generation application 610 and the initial active insulin amounts for the respective pharmacokinetics compartments to determine an updated amount of active insulin for each of the respective pharmacokinetics compartments. Thereafter, the active insulin application 612 calculates the total active insulin amount based on the amount of insulin delivered minus the active insulin amount for the effect-site compartment. The active insulin application 612 may store or otherwise maintain the closed-loop delivery data along with the active insulin amounts for the respective pharmacokinetics compartments in memory 606 to support iteratively and recursively calculating an updated total active insulin amount upon each iteration of the loop defined by tasks 804, 806, 808, 810, 812 and 814 of the active insulin notification process 800.

In exemplary embodiments, the active insulin amounts for the respective pharmacokinetics compartments in the closed-loop operating mode are calculated using the following equation:

$$\begin{bmatrix} I_p(k) \\ I_s(k) \\ I_e(k) \end{bmatrix} = \begin{bmatrix} A_{11} & A_{12} & 0 \\ 0 & A_{22} & 0 \\ A_{31} & 0 & A_{33} \end{bmatrix} \begin{bmatrix} I_p(k-1) \\ I_s(k-1) \\ I_e(k-1) \end{bmatrix} + \begin{bmatrix} B_1 \\ B_2 \\ 0 \end{bmatrix} u(k)$$, where $I_p(k)$ represents the current amount of insulin in the

plasma compartment, $I_s(k)$ represents the current amount of insulin in the subcutaneous compartment, $I_e(k)$ represents the current amount of insulin in the effect-site compartment, $u(k)$ represents the current (or most recent) dosage amount, and $I_p(k$-1), $I_s(k$-1), and $I_e(k$-1) are the amounts of insulin in the respective compartments from the preceding iteration. Thus, for an initial iteration ($k$=1), the current amount of insulin in the plasma compartment ($I_p(1)$) is equal to $A_{11}I_p(0) + A_{12}I_s(0) + B_1u(1)$, where $I_p(0)$ and $I_s(0)$ are the initial active insulin in the plasma and subcutaneous compartments, respectively, upon entering the closed-loop mode (e.g., from task 802) and $u(1)$ is the amount of insulin delivered in the first basal delivery (e.g., the amount of insulin corresponding to the initial closed-loop basal dosage command determined by the command generation application 610). Similarly, the current amount of active insulin in the effect-site compartment for the first iteration ($I_e(1)$) is equal to $A_{31}I_p(0) + A_{33}I_e(0)$, where $I_p(0)$ and $I_e(0)$ are the initial active insulin in the plasma and effect-site compartments upon entering the closed-loop mode.

As described above, it should be noted that the $u(k)$ term varies according to the variable basal rate implemented by the command generation application 610. Additionally, the $u(k)$ term includes any manually-initiated bolus amounts that were delivered during the closed-loop operating mode, which may be superimposed over the basal rate dosage command at a particular iteration (k) or administered in lieu of the basal rate dosage command at that particular iteration. After determining the current amount of insulin in the effect-site compartment, the current total amount of active insulin ($T_I(k)$) is calculated using the equation $T_I(k) = \sum_{i=1}^{k} u(i) - \sum_{i=1}^{k} I_e(i)$. As described above, the active insulin application 612 may store or otherwise maintain the closed-loop delivery data (e.g., the values for $u(k)$ along with the insulin amounts for the effect-site compartment to support iteratively and recursively calculating an updated total active insulin amount $T_I(k)$ upon each iteration of the loop defined by tasks 804, 806, 808, 810, 812 and 814 of the active insulin notification process 800.

The $A_{11}, A_{12}, A_{22}, A_{31}, A_{33}, B_1$, and $B_2$, terms represent absorption coefficients for the respective compartments. In one or more embodiments, the coefficients may be governed by the following equations:

$$A_{11} = e^{-T_s/50}, \quad A_{12} = 2.5\left(e^{-T_s/70} - e^{-T_s/50}\right), \quad A_{22} = e^{-T_s/70}, \quad A_{31} = T_s/55, \quad A_{33} = -T_s/70, \quad B_1 = 60\left(1 - e^{-T_s/50}\right),$$

and $B_2 = 3\left[70\left(1 - e^{-T_s/70}\right) - 50\left(1 - e^{-T_s/50}\right)\right]$, where $T_s$ the sampling time (in minutes) associated with the closed-loop operating mode. In this regard, $T_s$ corresponds to the difference in time between successive closed-loop basal dosage commands generated by the command generation application 610.

Referring again to FIG. 8, the active insulin notification process 800 also recursively calculates or otherwise determines a nominal amount of active insulin in the body of the patient based on a reference basal delivery rate (task 812). The nominal amount of active insulin represents the amount of active insulin that is expected to bring the patient's glucose measurements to a substantially constant or stable fasting glucose value, which, in some embodiments, may be equal to the target glucose value referenced by the closed-loop control system 700. In exemplary embodiments, the reference basal delivery rate ($v(k)$) is calculated or otherwise determined based on the patient's total daily dose and the sampling time. The reference basal delivery rate may be governed by the equation $v(k) = \frac{TDD}{48} \times \frac{T_s}{60}$, where $TDD$ represents a patient-specific total daily dose and $T_s$ is the sampling time associated with the closed-loop operating mode as described above. In one or more embodiments, the patient-specific total daily dose is determined based on historical delivery information over a preceding interval of time (e.g., the amount of insulin delivered by the infusion device 502 over the preceding 24 hours). In this regard, the total amount of insulin delivered by the infusion device 502 over the preceding interval may be stored or otherwise maintained in the memory 606 of the infusion device 502 and dynamically updated over time. In other embodiments, the total daily dose may be a configurable user setting that is manually set to a fixed value by the patient or other user via a user interface element 540, 608, and then stored as a patient setting in the memory 606.

In exemplary embodiments, after determining the reference basal delivery rate based on the patient's total daily dose, the active insulin application 612 iteratively and recursively determines the current nominal active insulin amounts for the respective pharmacokinetics compartments using the equation:

$$\begin{bmatrix} I_{Np}(k) \\ I_{Ns}(k) \\ I_{Ne}(k) \end{bmatrix} = \begin{bmatrix} A_{11} & A_{12} & 0 \\ 0 & A_{22} & 0 \\ A_{31} & 0 & A_{33} \end{bmatrix} \begin{bmatrix} I_{Np}(k-1) \\ I_{Ns}(k-1) \\ I_{Ne}(k-1) \end{bmatrix} + \begin{bmatrix} B_1 \\ B_2 \\ 0 \end{bmatrix} v(k)$$

. In this regard, the set of coefficient variables used in calculating nominal insulin amounts in the respective pharmacokinetics compartments are identical to the coefficient variables used in calculating the current active insulin amounts in the respective pharmacokinetics compartments. As described above, it should be noted that the $v(k)$ term is constant (or fixed) and corresponds to the patient's total daily dose for achieving a desired fasting glucose level. After determining the current nominal amount of insulin in the effect-site compartment, the current nominal amount of active insulin ($N_I(k)$) is calculated using the equation

$$N_I(k) = \sum_{i=1}^{k} v(i) - \sum_{i=1}^{k} I_{Ne}(i)$$

.

By way of example, for an initial iteration (k=1), the nominal amount of active insulin in the plasma compartment ($I_{Np}(1)$) is equal to $A_{11}I_{Np}(0) + A_{12}I_{Ns}(0) + B_1v(1)$, where $I_{Np}(0)$ and $I_{Ns}(0)$ are the initial active insulin in the plasma and subcutaneous compartments, respectively, upon entering the closed-loop mode (e.g., $I_p(0) = I_{Np}(0)$) and $v(1)$ is the amount of insulin that would be delivered in each basal delivery according to the reference basal rate of infusion. Similarly, the nominal amount of active insulin in the effect-site compartment for the first iteration ($I_{Ne}(1)$) is equal to $A_{31}I_{Np}(0) + A_{33}I_{Ne}(0)$, where $I_{Ne}(0) = I_e(0)$, and the nominal amount of active insulin is equal to $v(1)$-$I_{Ne}(1)$.

The loop defined by tasks 804, 806, 808, 810, 812 and 814 of the active insulin notification process 800 repeats during operation of the infusion device 502 in the closed-loop operating mode to dynamically vary the basal infusion rate based on updated glucose measurements from the sensing arrangement 504 to autonomously regulate the patient's glucose level to a reference glucose level, and iteratively and recursively calculate the current total amount of active insulin ($T_I(k)$) and the current nominal amount of active insulin ($N_I(k)$). Thus, for a second iteration (k=2), the current amount of insulin in the plasma compartment ($I_p(2)$) is equal to $A_{11}I_p(1) + A_{12}I_s(1) + B_1u(2)$, where $u(2)$ is the amount of insulin delivered in the second basal delivery. It should be noted that the preceding instance of the amount of insulin in the subcutaneous compartment ($I_s(1)$) is equal to $A_{22}I_s(0) + B_2u(1)$, and thus, is also influenced by the variable basal rate. The current amount of insulin in the effect-site compartment for the second iteration ($I_e(2)$) is equal to $A_{31}I_p(1) + A_{33}I_e(1)$, where $I_p(1)$ and $I_e(1)$ are the preceding instances of the insulin in the plasma and effect-site compartments, and the current total amount of active insulin for the second iteration is determined by $T_I(2) = (u(1) + u(2)) - (I_e(1) + I_e(2))$.

Similarly, the nominal amount of active insulin in the plasma compartment for the second iteration ($I_{Np}(2)$) is equal to $A_{11}I_{Np}(1) + A_{12}I_{Ns}(1) + B_1v(2)$, the nominal amount of insulin in the effect-site compartment for the second iteration is equal to $A_{31}I_{Np}(1) + A_{33}I_{Ne}(1)$, and the current nominal amount of active insulin for the second iteration is determined as $N_I(2) = (v(1) + v(2)) - (I_{Ne}(1) + I_{Ne}(2))$. Again, it should be noted that $v(2) = v(1)$, because $v(k)$ is constant.

As illustrated in FIG. 8, in response to detecting or otherwise identifying termination of the closed-loop operating mode, the active insulin notification process 800 calculates or otherwise determines the current residual amount of active insulin based on the current total amount of active insulin and the current nominal amount of active insulin and generates or otherwise provides a graphical representation of the current residual active insulin (tasks 814, 816, 818). In one or more embodiments, the patient or another user manipulates a user interface element 540, 608 of the infusion device 502 to manually exit the closed-loop operating mode and transition to another operating mode, such as an open-loop operating mode or a manual operating mode. That said, in some embodiments, the closed-loop operating mode may automatically terminate or exit (e.g., by timing out or otherwise reaching a maximum allowed duration, based on a glucose measurement value, or the like). The pump control system 520, 600 of the infusion device 502 may automatically determine the destination operating mode to transition to, for example, as described in U.S. Patent Application Serial No. 14/561,133.

In exemplary embodiments, the active insulin application 612 determines the residual amount of active insulin by subtracting the current nominal amount of active insulin from the current total amount of active insulin. In this regard, the residual amount of active insulin corresponds to the current active insulin that is in excess of the estimated amount of active insulin expected to produce the desired fasting glucose level. In exemplary embodiments, the residual insulin is bounded so that is nonnegative, for example, using the equation $R_I(k) = \max(0, T_I(k) - N_I(k))$, where $R_I(k)$ represents the current residual active insulin. Thus, if the closed-loop operating mode is exited after the second iteration, the current residual active insulin may be represented by the equation $R_I(2) = \max(0, T_I(2) - N_I(2))$. It should be appreciated that as the variable basal rate of infusion ($u(k)$) varies with respect to the reference basal rate of infusion ($v(k)$), the residual active insulin varies in a corresponding manner as influenced by the variable coefficient values ($A_{11}$, $A_{12}$, $A_{22}$, $A_{31}$, $A_{33}$, $B_1$, and $B_2$).

The active insulin application 612 generates or otherwise provides a graphical representation of the current residual active insulin on a user interface element 540, 608 associated with the infusion device 502 (e.g., display 226), thereby apprising the patient of the current amount of insulin-on-board that exceeds the expected amount of insulin required to achieve a desired steady-state fasting glucose level corresponding to the patient's total daily dose. Thus, upon transitioning to another operating mode, the patient may readily ascertain the current state of the insulin in his or her body and determine whether any actions should be performed to account for the residual amount of active insulin. For example, if the patient is about to consume a meal, the patient may determine he or she can forgo a meal bolus based on the residual amount of active insulin exceeding the meal bolus amount the patient would otherwise administer. Conversely, if the patient is about to engage in exercise, the patient may determine he or she should consume carbohydrates first based on the residual amount of active insulin being relatively low (or less than desired).

In the illustrated embodiment, the active insulin notification process 800 identifies or otherwise determines a recommended remedial action based on the current residual active insulin and generates or otherwise provides an indication of the recommended remedial action (tasks 820, 822). Based on the magnitude of the current residual active insulin amount, the active insulin application 612 may determine one or more remedial actions that should be performed by the patient to mitigate the effects of the residual insulin and display the recommended remedial action(s) on a display 226, 540, 608 associated with the infusion device 502. For example, if the current residual active insulin amount is greater than a threshold value, the active insulin application 612 may determine that the patient should consume carbohydrates to prevent a potential hypoglycemic condition. The active insulin application 612 may convert the difference between the current residual active insulin amount and the threshold residual active insulin value to a corresponding amount of carbohydrates, which may then be displayed or otherwise indicated on the display 226, 540, 608. That said, when the infusion device 502 transitions from an autonomous closed-loop operating mode to another operating mode due to an anomalous condition with respect to the autonomous operation of the infusion device 502, the active insulin notification process 800 may forego providing recommended actions to the patient (e.g., by skipping tasks 820 and 822) and simply display the residual insulin amount and allow the patient to best determine how to proceed in view of the anomalous condition.

To briefly summarize, the subject matter described herein allows for a patient to be apprised of the current amount of active insulin when transitioning from an autonomous operating mode in a manner that allows the patient to readily ascertain how to proceed managing his or her glycemic state. The residual active insulin amount determined based on the variable basal rate of infusion provided in accordance with a closed-loop operating mode (or another autonomous operating mode) relative to a reference basal rate of infusion provides an accurate picture of the patient's current active insulin that allows the patient or another user to readily ascertain what, if any, actions are required. Moreover, in some embodiments, the residual active insulin amount may be utilized to automatically identify recommended remedial actions and provide corresponding notifications to the patient, thereby further aiding the patient in managing his or her condition.

In one or more embodiments, an "autonomous operating mode," "autonomously operating," and variants thereof refer to an infusion device implementing an operating mode where the infusion device is capable of automatically delivering fluid in an automated manner independent of and without the need for any concurrent manual interaction or other external influence other than the input(s) to the automated control algorithm(s) described herein (e.g., sensor data, measurement data, and/or the like).

For the sake of brevity, conventional techniques related to glucose sensing and/or monitoring, closed-loop glucose control, and other functional aspects of the subject matter may not be described in detail herein. In addition, certain terminology may also be used in the herein for the purpose of reference only, and thus is not intended to be limiting. For example, terms such as "first", "second", and other such numerical terms referring to structures do not imply a sequence or order unless clearly indicated by the context. The foregoing description may also refer to elements or nodes or features being "connected" or "coupled" together. As used herein, unless expressly stated otherwise, "coupled" means that one element/node/feature is directly or indirectly joined to (or directly or indirectly communicates with) another element/node/feature, and not necessarily mechanically.

While at least one exemplary embodiment has been presented in the foregoing detailed description, it should be appreciated that a vast number of variations exist. It should also be appreciated that the exemplary embodiment or embodiments described herein are not intended to limit the scope, applicability, or configuration of the claimed subject matter in any way. For example, the subject matter described herein is not necessarily limited to the infusion devices and related systems described herein. Moreover, the foregoing detailed description will provide those skilled in the art with a convenient road map for implementing the described embodiment or embodiments. It should be understood that various changes can be made in the function and arrangement of elements without departing from the scope defined by the claims. Accordingly, details of the exemplary embodiments or other limitations described above should not be read into the claims absent a clear intention to the contrary.

## Claims

1. A method of operating an infusion device (102) having a motor (507) operable to deliver a fluid to a body of a user and a control system (500) coupled to the motor, and capable of delivering an active fluid to a body of a user in one of at least two operating modes and which can change between said operating modes, the method comprising:

monitoring the rate of infusion of said active fluid delivered by said infusion device (102) in a first operating mode (804, 806, 808, 810, 812, 814) and determining a residual amount of active fluid in the body of the user; and

in response to identifying a change in operating mode from the first operating mode to a second operating mode, generating, by the control system (500), a user notification (818) based at least in part on the residual amount of active fluid.

2. An infusion device configured to operate in accordance with the method of claim 1.

3. The infusion device of claim 2, wherein determining the residual amount of active fluid comprises:

determining a total amount of active fluid in the body of the user based at least in part on the monitored rate of infusion;

determining a nominal amount of active fluid in the body of the user based at least in part on a reference rate of infusion; and

determining the residual amount based on a difference between the total amount and the nominal amount.

4. The infusion device of claim 3, wherein determining the nominal amount comprises determining the reference rate of infusion based on a total daily dose of the fluid for the user.

5. The infusion device of claim 3, wherein:

determining the total amount of active fluid comprises iteratively determining a first amount of active fluid in a pharmacokinetics compartment based on the monitored rate of infusion and a first set of variables; and

determining the nominal amount of active fluid comprises iteratively determining a second amount of active fluid in the pharmacokinetics compartment based on the reference rate of infusion and the first set of variables.

6. The infusion device of claim 5, wherein:

determining the total amount of active fluid comprises subtracting a first cumulative amount of active fluid in the pharmacokinetics compartment determined based on the monitored rate of infusion from a second cumulative amount of fluid delivered based on the monitored rate of infusion; and

determining the nominal amount of active fluid comprises subtracting a third cumulative amount of active fluid in the pharmacokinetics compartment determined based on the reference rate of infusion from a fourth cumulative amount of fluid corresponding to the reference rate of infusion.

7. The infusion device of claim 5, wherein:

iteratively determining the first amount of active fluid comprises:

determining an active fluid amount for a plasma compartment based on the monitored rate of infusion, a preceding instance of the active fluid amount for the plasma compartment, and a first subset of the first set of variables; and

determining the first amount of active fluid for an effect-site compartment based at least in part on the preceding instance of the active fluid amount for the plasma compartment, a preceding instance of the first amount of active fluid for the effect-site compartment, and a second subset of the first set of variables; and

iteratively determining the second amount of active fluid comprises:

determining a second active fluid amount for the plasma compartment based on the reference rate of infusion, a preceding instance of the second active fluid amount for the plasma compartment, and the first subset of the first set of variables; and

determining the second amount of active fluid for the effect-site compartment based at least in part on the preceding instance of the second active fluid amount for the plasma compartment, a preceding instance of the second amount for the effect-site compartment, and the second subset of the first set of variables.

8. The infusion device of claim 2, wherein generating the user notification (818) comprises displaying a graphical representation of the residual amount of active fluid.

9. The infusion device of claim 2, wherein determining the residual amount of active fluid comprises determining the residual amount of active fluid based at least in part on the monitored rate of infusion and a total daily requirement of the fluid associated with the user.

10. The infusion device of any of claims 2 to 9, wherein the first operating mode is a closed-loop mode in which the infusion device is capable of delivering the active fluid based on measured values for a physiological condition in the body of the user, the physiological condition being influenced by the fluid delivered by the infusion device.

11. An infusion system (100) comprising:

a user interface (230);
an infusion device (102) including a motor (507) operable to deliver a fluid to a body of a user and a control system (500) coupled to the motor, the fluid being selected to influence a physiological condition of the user; and
a sensing arrangement (504) to obtain measurement values for the physiological condition from the body of the user, wherein the control system is coupled to the user interface (540) and the sensing arrangement and in a first operating mode is configured to operate the motor (507) to deliver a variable rate of infusion based on the measurement values;
wherein the control system (500) is further configured to determine a residual amount of the fluid that is active in the body of the user based at least in part on the variable rate of infusion, and
when exiting said first operating mode to provide a user notification on the user interface influenced by the residual amount.

12. The infusion system of claim 11, wherein the residual amount comprises a difference between a total amount of active fluid in the body of the user corresponding to the variable rate of infusion and a nominal amount of active fluid in the body of the user corresponding to a reference rate of infusion.

13. The infusion system of claim 12, wherein:

the infusion device includes a data storage element (606) maintaining a total daily dose for the user; and
the control system is coupled to the data storage element and determines the reference rate of infusion based on the total daily dose.

14. The infusion system of claim 13, wherein:

the infusion device includes a data storage element (606) maintaining a total daily dose for the user; and
the control system is coupled to the data storage element and determines the residual amount based at least in part on the total daily dose and the variable rate of infusion.

15. The infusion device of claim 10 or the infusion system of any of claims 11 to 14, wherein the physiological condition of the user is the user's blood glucose level and the fluid is insulin.


**Patentansprüche**

1. Verfahren zum Betreiben einer Infusionsvorrichtung (102), die einen Motor (507), der betreibbar ist, um ein Fluid an einen Körper eines Benutzers abzugeben, und ein Steuerungssystem (500) aufweist, das an den Motor gekoppelt und fähig ist, ein aktives Fluid an den Körper eines Benutzers in einem von mindestens zwei Betriebsmodi abzugeben, und das zwischen den Betriebsmodi wechseln kann, das Verfahren umfassend:

Überwachen der Infusionsrate das aktiven Fluids, das durch die Infusionsvorrichtung (102) in einem ersten Betriebsmodus (804, 806, 808, 810, 812, 814) abgegeben wird, und Bestimmen einer Restmenge an aktivem Fluid in dem Körper des Benutzers; und
als Reaktion auf das Identifizieren einer Änderung des Betriebsmodus von dem ersten Betriebsmodus zu einem zweiten Betriebsmodus, Erzeugen, durch das Steuerungssystem (500), einer Benutzerbenachrichtigung (818) basierend mindestens teilweise auf der Restmenge an aktivem Fluid.

2. Infusionsvorrichtung, die konfiguriert ist, um gemäß dem Verfahren nach Anspruch 1 zu arbeiten.

3. Infusionsvorrichtung nach Anspruch 2, wobei das Bestimmen der Restmenge an aktivem Fluid umfasst:

Bestimmen einer Gesamtmenge an aktivem Fluid in dem Körper des Benutzers basierend mindestens teilweise auf der überwachten Infusionsrate;
Bestimmen einer Nominalmenge an aktivem Fluid in dem Körper des Benutzers basierend mindestens teilweise auf einer Referenzinfusionsrate; und
Bestimmung der Restmenge basierend auf einer Differenz zwischen der Gesamtmenge und der Nominalmenge.

4. Infusionsvorrichtung nach Anspruch 3, wobei das Bestimmen der Nominalmenge das Bestimmen der Referenzinfusionsrate basierend auf einer täglichen Gesamtdosis des Fluids für den Benutzer umfasst.

5. Infusionsvorrichtung nach Anspruch 3, wobei:

das Bestimmen der Gesamtmenge an aktivem Fluid das iterative Bestimmen einer ersten Menge an aktivem Fluid in einem pharmakokinetischen Kompartiment basierend auf der überwachten Infusionsrate und einem ersten Satz von Variablen umfasst; und
das Bestimmen der Nominalmenge an aktivem Fluid das iterative Bestimmen einer zweiten Menge an aktivem Fluid in dem pharmakokinetischen Kompartiment basierend auf der Referenzinfusionsrate und dem ersten Satz von Variablen umfasst.

6. Infusionsvorrichtung nach Anspruch 5, wobei:

das Bestimmen der Gesamtmenge an aktivem Fluid das Subtrahieren einer ersten kumulativen Menge an aktivem Fluid in dem pharmakokinetischen Kompartiment, die basierend auf der überwachten Infusionsrate aus einer zweiten kumulativen Menge an abgegebenem Fluid bestimmt wird, die basierend auf der überwachten Infusionsrate bestimmt wird, umfasst; und
das Bestimmen der nominalen Menge an aktivem Fluid das Subtrahieren einer dritten kumulativen Menge an aktivem Fluid in dem pharmakokinetischen Kompartiment, die basierend auf der Referenzinfusionsrate von einer vierten kumulativen Fluidmenge bestimmt wird, die der Referenzinfusionsrate entspricht, umfasst.

7. Infusionsvorrichtung nach Anspruch 5, wobei:
das iterative Bestimmen der ersten Menge an aktivem Fluid umfasst:

Bestimmen einer aktiven Fluidmenge für ein Plasmakompartiment basierend auf der überwachten Infusionsrate, einer vorangehenden Instanz der aktiven Fluidmenge für das Plasmakompartiment und einer ersten Teilmenge der ersten Menge an Variablen; und
Bestimmen der ersten Menge an aktivem Fluid für ein Wirkstellenkompartiment basierend mindestens teilweise auf der vorangehenden Instanz der aktiven Fluidmenge für das Plasmakompartiment, einer vorangehenden Instanz der ersten Menge an aktivem Fluid für das Wirkstellenkompartiment und einer zweiten Teilmenge der ersten Menge an Variablen; und
das iterative Bestimmen der zweiten Menge an aktivem Fluid umfasst:

Bestimmen einer zweiten aktiven Fluidmenge für das Plasmakompartiment basierend auf der Referenzinfusionsrate, einer vorangehenden Instanz der zweiten aktiven Fluidmenge für das Plasmakompartiment und der ersten Teilmenge des ersten Satzes von Variablen; und
Bestimmen der zweiten Menge an aktivem Fluid für das Wirkstellenkompartiment basierend mindestens teilweise auf der vorangehenden Instanz der zweiten aktiven Fluidmenge für das Plasmakompartiment, einer vorangehenden Instanz der zweiten Menge für das Wirkstellenkompartiment und der zweiten Teilmenge des ersten Satzes von Variablen.

8. Infusionsvorrichtung nach Anspruch 2, wobei das Erzeugen der Benutzerbenachrichtigung (818) ein Anzeigen einer graphischen Darstellung der Restmenge an aktivem Fluid umfasst.

9. Infusionsvorrichtung nach Anspruch 2, wobei das Bestimmen der Restmenge an aktivem Fluid das Bestimmen der Restmenge an aktivem Fluid basierend mindestens teilweise auf der überwachten Infusionsrate und einem täglichen Gesamtbedarf des mit dem Benutzer assoziierten Fluids umfasst.

10. Infusionsvorrichtung nach einem der Ansprüche 2 bis 9, wobei der erste Betriebsmodus ein geschlossener Regelkreismodus ist, in dem die Infusionsvorrichtung fähig ist, das aktive Fluid basierend auf gemessenen Werten für einen physiologischen Zustand in dem Körper des Benutzers abzugeben, wobei der physiologische Zustand durch das von die Infusionsvorrichtung abgegebene Fluid beeinflusst wird.

11. Infusionssystem (100), umfassend:

eine Benutzerschnittstelle (230);
eine Infusionsvorrichtung (102), die einen Motor (507) einschließt, der betreibbar ist, um ein Fluid an den Körper eines Benutzers abzugeben, und ein Steuerungssystem (500), das an den Motor gekoppelt ist, wobei das Fluid ausgewählt ist, um einen physiologischen Zustand des Benutzers zu beeinflussen; und
eine Abtastanordnung (504), um Messwerte für den physiologischen Zustand von dem Körper des Benutzers zu erhalten, wobei das Steuerungssystem an die Benutzerschnittstelle (540) und die Abtastanordnung gekoppelt ist und in einem ersten Betriebsmodus konfiguriert ist, um den Motor (507) zu betreiben, um eine variable Infusionsrate basierend auf den Messwerten abzugeben;
wobei das Steuerungssystem (500) ferner konfiguriert ist, um eine Restmenge des Fluids zu bestimmen, das in dem Körper des Benutzers aktiv ist, basierend mindestens teilweise auf der variablen Infusionsrate, und beim Verlassen des ersten Betriebsmodus eine Benutzerbenachrichtigung auf der Benutzerschnittstelle bereitzustellen, die durch die Restmenge beeinflusst wird.

12. Infusionssystem nach Anspruch 11, wobei die Restmenge eine Differenz zwischen einer Gesamtmenge an aktivem Fluid in dem Körper des Benutzers entsprechend der variablen Infusionsrate und einer Nominalmenge an aktivem Fluid in dem Körper des Benutzers entsprechend einer Referenzinfusionsrate umfasst.

13. Infusionssystem nach Anspruch 12, wobei:

die Infusionsvorrichtung ein Datenspeicherelement (606) einschließt, das eine tägliche Gesamtdosis für den Benutzer aufrechterhält; und
das Steuerungssystem an das Datenspeicherelement gekoppelt ist und die Referenzinfusionsrate basierend auf der täglichen Gesamtdosis bestimmt.

14. Infusionssystem nach Anspruch 13, wobei:

die Infusionsvorrichtung ein Datenspeicherelement (606) einschließt, das eine tägliche Gesamtdosis für den Benutzer aufrechterhält; und
das Steuerungssystem an das Datenspeicherelement gekoppelt ist und die Restmenge mindestens teilweise basierend auf der täglichen Gesamtdosis und der variablen Infusionsrate bestimmt.

15. Infusionsvorrichtung nach Anspruch 10 oder das Infusionssystem nach einem der Ansprüche 11 bis 14, wobei der physiologische Zustand des Benutzers der Blutzuckerspiegel des Benutzers ist und das Fluid Insulin ist.

## Revendications

1. Procédé de fonctionnement d'un dispositif de perfusion (102) ayant un moteur (507) pouvant être actionné pour administrer un fluide à un corps d'un utilisateur et un système de contrôle (500) couplé au moteur, et capable d'administrer un fluide actif à un corps d'un utilisateur dans l'un parmi au moins deux modes de fonctionnement et qui peut basculer entre lesdits modes de fonctionnement, le procédé comprenant :

la surveillance du débit de perfusion dudit fluide actif administré par ledit dispositif de perfusion (102) dans un premier mode de fonctionnement (804, 806, 808, 810, 812, 814) et la détermination d'une quantité résiduelle de fluide actif dans le corps de l'utilisateur ; et
en réponse à l'identification d'un changement de mode de fonctionnement du premier mode de fonctionnement vers un second mode de fonctionnement, la génération, par le système de contrôle (500), d'une notification utilisateur (818) en fonction au moins en partie de la quantité résiduelle de fluide actif.

2. Dispositif de perfusion configuré pour fonctionner conformément au procédé selon la revendication 1.

3. Dispositif de perfusion selon la revendication 2, dans lequel la détermination de la quantité résiduelle de fluide actif comprend :

la détermination d'une quantité totale de fluide actif dans le corps de l'utilisateur en fonction au moins en partie du débit de perfusion surveillé ;

la détermination d'une quantité nominale de fluide actif dans le corps de l'utilisateur en fonction au moins en partie d'un débit de perfusion de référence ; et

la détermination de la quantité résiduelle en fonction d'une différence entre la quantité totale et la quantité nominale.

4. Dispositif de perfusion selon la revendication 3, dans lequel la détermination de la quantité nominale comprend la détermination du débit de perfusion de référence en fonction d'une dose quotidienne totale du fluide pour l'utilisateur.

5. Dispositif de perfusion selon la revendication 3, dans lequel :

la détermination de la quantité totale de fluide actif comprend la détermination itérative d'une première quantité de fluide actif dans un compartiment pharmacocinétique en fonction du débit de perfusion surveillé et d'un premier ensemble de variables ; et

la détermination de la quantité nominale de fluide actif comprend la détermination itérative d'une seconde quantité de fluide actif dans le compartiment pharmacocinétique en fonction du débit de perfusion de référence et du premier ensemble de variables.

6. Dispositif de perfusion selon la revendication 5, dans lequel :

la détermination de la quantité totale de fluide actif comprend la soustraction d'une première quantité cumulée de fluide actif dans le compartiment pharmacocinétique déterminée en fonction du débit de perfusion surveillé d'une deuxième quantité cumulée de fluide administrée en fonction du débit de perfusion surveillé ; et

la détermination de la quantité nominale de fluide actif comprend la soustraction d'une troisième quantité cumulée de fluide actif dans le compartiment pharmacocinétique déterminé en fonction du débit de perfusion de référence d'une quatrième quantité cumulée de fluide correspondant au débit de perfusion de référence.

7. Dispositif de perfusion selon la revendication 5, dans lequel :
la détermination itérative de la première quantité de fluide actif comprend :

la détermination d'une quantité de fluide actif pour un compartiment plasmatique en fonction du débit de perfusion surveillé, d'une instance précédente de la quantité de fluide actif pour le compartiment plasmatique, et d'un premier sous-ensemble du premier ensemble de variables ; et

la détermination de la première quantité de fluide actif pour un compartiment de site d'effet en fonction au moins en partie de l'instance précédente de la quantité de fluide actif pour le compartiment plasmatique, d'une instance précédente de la première quantité de fluide actif pour le compartiment de site d'effet, et d'un second sous-ensemble du premier ensemble de variables ; et

la détermination itérative de la seconde quantité de fluide actif comprend :

la détermination d'une seconde quantité de fluide actif pour le compartiment plasmatique en fonction du débit de perfusion de référence, d'une instance précédente de la seconde quantité de fluide actif pour le compartiment plasmatique, et du premier sous-ensemble du premier ensemble de variables ; et

la détermination de la seconde quantité de fluide actif pour le compartiment de site d'effet en fonction au moins en partie de l'instance précédente de la seconde quantité de fluide actif pour le compartiment plasmatique, d'une instance précédente de la seconde quantité pour le compartiment de site d'effet, et du second sous-ensemble du premier ensemble de variables.

8. Dispositif de perfusion selon la revendication 2, dans lequel la génération de la notification utilisateur (818) comprend l'affichage d'une représentation graphique de la quantité résiduelle de fluide actif.

9. Dispositif de perfusion selon la revendication 2, dans lequel la détermination de la quantité résiduelle de fluide actif comprend la détermination de la quantité résiduelle de fluide actif en fonction au moins en partie du débit de perfusion surveillé et d'un besoin quotidien total du fluide associé à l'utilisateur.

10. Dispositif de perfusion selon l'une quelconque des revendications 2 à 9, dans lequel le premier mode de fonctionnement est un mode en boucle fermée dans lequel le dispositif de perfusion est capable d'administrer le fluide actif en fonction de valeurs mesurées pour un état physiologique dans le corps de l'utilisateur, l'état physiologique étant influencé par le fluide administré par le dispositif de perfusion.

11. Système de perfusion (100) comprenant :

une interface utilisateur (230) ;
un dispositif de perfusion (102) comportant un moteur (507) pouvant être actionné pour administrer un fluide à un corps d'un utilisateur et un système de contrôle (500) couplé au moteur, le fluide étant choisi pour influencer un état physiologique de l'utilisateur ; et
un agencement de détection (504) pour obtenir des valeurs de mesure pour l'état physiologique à partir du corps de l'utilisateur, dans lequel le système de contrôle est couplé à l'interface utilisateur (540) et à l'agencement de détection et, dans un premier mode de fonctionnement, est configuré pour actionner le moteur (507) afin d'administrer un débit de perfusion variable en fonction des valeurs de mesure ;
dans lequel le système de contrôle (500) est en outre configuré pour déterminer une quantité résiduelle du fluide qui est actif dans le corps de l'utilisateur en fonction au moins en partie du débit de perfusion variable, et lors de la sortie dudit premier mode de fonctionnement pour fournir une notification utilisateur sur l'interface utilisateur influencée par la quantité résiduelle.

12. Système de perfusion selon la revendication 11, dans lequel la quantité résiduelle comprend une différence entre une quantité totale de fluide actif dans le corps de l'utilisateur correspondant au débit de perfusion variable et une quantité nominale de fluide actif dans le corps de l'utilisateur correspondant à un débit de perfusion de référence.

13. Système de perfusion selon la revendication 12, dans lequel :

le dispositif de perfusion comporte un élément de stockage de données (606) maintenant une dose quotidienne totale pour l'utilisateur ; et
le système de contrôle est couplé à l'élément de stockage de données et détermine le débit de perfusion de référence en fonction de la dose quotidienne totale.

14. Système de perfusion selon la revendication 13, dans lequel :

le dispositif de perfusion comporte un élément de stockage de données (606) maintenant une dose quotidienne totale pour l'utilisateur ; et
le système de contrôle est couplé à l'élément de stockage de données et détermine la quantité résiduelle en fonction au moins en partie de la dose quotidienne totale et du débit de perfusion variable.

15. Dispositif de perfusion selon la revendication 10 ou système de perfusion selon l'une quelconque des revendications 11 à 14, dans lequel l'état physiologique de l'utilisateur est la glycémie de l'utilisateur et le fluide est l'insuline.

FIG. 1

FIG. 2

FIG. 3

FIG. 4

FIG. 5

FIG. 6

EP 3 297 705 B1

FIG. 7

ACTIVE INSULIN NOTIFICATION PROCESS ⟩—800

DETERMINE INITIAL ACTIVE INSULIN BASED ON MANUAL BOLUSES —802

OBTAIN CURRENT GLUCOSE MEASUREMENT VALUE —804

OPERATE INFUSION DEVICE IN CLOSED-LOOP OPERATING MODE BASED ON CURRENT GLUCOSE MEASUREMENT VALUE —806

OBTAIN CLOSED-LOOP DELIVERY DATA —808

RECURSIVELY DETERMINE TOTAL ACTIVE INSULIN USING CLOSED-LOOP DELIVERY DATA —810

RECURSIVELY DETERMINE NOMINAL ACTIVE INSULIN BASED ON REFERENCE BASAL DELIVERY RATE —812

EXIT CLOSED-LOOP ? 814

NO

YES

DETERMINE RESIDUAL ACTIVE INSULIN BASED ON TOTAL ACTIVE INSULIN AND NOMINAL ACTIVE INSULIN —816

DISPLAY RESIDUAL ACTIVE INSULIN TO USER —818

DETERMINE RECOMMENDED ACTION BASED ON RESIDUAL ACTIVE INSULIN —820

PROVIDE INDICATION OF RECOMMENDED ACTION —822

EXIT

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015191459 A
- US 20100295686 A1
- US 20100094251 A
- WO 2009133558 A
- US 20160158441 A
- US 4562751 A
- US 4685903 A
- US 5080653 A
- US 5505709 A
- US 5097122 A
- US 6485465 B
- US 6554798 B
- US 6558320 B
- US 6558351 B
- US 6641533 B
- US 6659980 B
- US 6752787 B
- US 6817990 B
- US 6932584 B
- US 7621893 B
- US 8674288 B
- US 6088608 A
- US 6119028 A
- US 6589229 B
- US 6740072 B
- US 6827702 B
- US 7323142 B
- US 7402153 B
- US 6248093 B
- US 8474332 B
- US 561133